# EUROPEAN PATENT SPECIFICATION

(11) **EP 3 943 933 B1**
(45) Date of publication and mention of the grant of the patent: **09.12.2026**
(21) Application number: 20778791.2
(22) Date of filing: 17.03.2020
(51) Int. Cl.: G01N 33/48, C12M 1/34, C12Q 1/04, G01N 33/49, G01N 15/02, G01N 15/10, G01N 15/14, G01N 15/00, G06N 3/04, G06N 3/08

(54) **CELL ANALYSIS METHOD, TRAINING METHOD FOR DEEP LEARNING ALGORITHM, CELL ANALYSIS DEVICE, TRAINING METHOD FOR DEEP LEARNING ALGORITHM, CELL ANALYSIS PROGRAM, AND TRAINING PROGRAM FOR DEEP LEARNING ALGORITHM**
ZELLENANALYSEVERFAHREN, TRAININGSVERFAHREN FÜR EINEN TIEFENLERNALGORITHMUS, ZELLENANALYSEGERÄT, TRAININGSVERFAHREN FÜR EINEN TIEFENLERNALGORITHMUS, ZELLENANALYSEPROGRAMM UND TRAININGSPROGRAMM FÜR EINEN TIEFEN LERNALGORITHMUS
PROCÉDÉ D'ANALYSE DE CELLULE, PROCÉDÉ D'APPRENTISSAGE POUR ALGORITHME D'APPRENTISSAGE PROFOND, DISPOSITIF D'ANALYSE DE CELLULE, PROCÉDÉ D'APPRENTISSAGE POUR ALGORITHME D'APPRENTISSAGE PROFOND, PROGRAMME D'ANALYSE DE CELLULE ET PROGRAMME D'APPRENTISSAGE POUR ALGORITHME D'APPRENTISSAGE PROFOND

(30) Priority: 22.03.2019 JP 2019055385
(43) Date of publication of application: 26.01.2022
(73) Proprietor: SYSMEX CORPORATION, Kobe-shi Hyogo 651-0073 (JP)
(72) Inventor: KIMURA Konobu, Kobe-shi, Hyogo 651-0073 (JP); TANAKA Masamichi, Kobe-shi, Hyogo 651-0073 (JP); ASADA Shoichiro, Kobe-shi, Hyogo 651-2271 (JP)
(74) Representative: Hoffmann Eitle
(86) International application number: PCT/JP2020/011596
(87) International publication number: WO 2020/196074

(56) References cited:
- EP-A1- 3 073 265
- WO-A1-2017/073737
- WO-A1-2018/039216
- WO-A1-2018/203568
- WO-A1-2018/203568
- JP-A- 2001 174 456
- JP-A- 2014 106 059
- JP-A- 2016 186 463
- JP-A- 2017 219 479
- US-A1- 2017 052 106
- US-A1- 2018 286 038
- ROOPA B.HEGDE , KEERTHANA PRASAD , HARISHCHANDRA HEBBAR , BRIJ MOHAN KUMA SINGH: "Comparison of traditional image processing and deep learning approaches for classification of white blood cells in peripheral blood smear images", BIOCYBERNETICS AND BIOMEDICAL ENGINEERING, vol. 39, no. 2, 1 April 2019 (2019-04-01), pages 382 - 392, XP055856156, ISSN: 0208-5216, DOI: 10.1016/j.bbe.2019.01.005
- ROY, R. ET AL.: "Classification of WBC using deep learning for diagnosing diseases", PROCEEDINGS OF THE 2ND INTERNATIONAL CONFERENCE ON INVENTIVE COMMUNICATION AND COMPUTATIONAL TECHNOLOGIES (ICICCT) 2018, 27 September 2018 (2018-09-27), pages 1634 - 1638, XP033408960, DOI: 10.1109/ICICCT.2018.8473028
- SHAFIQUE, S. ET AL.: "Acute lymphoblastic leukemia detection and classification of its subtypes using pretrained deep covolutional neural networks", TECHNOLOGY IN CANCER RESEARCH & TREATMENT, vol. 17, 27 September 2018 (2018-09-27), XP055743559, DOI: 10.1177/1533033818802789
- TOMOHIKO AI, YOKO TABE, HIROYUKI TAKEMURA, KONOBU KIMURA, TOSHIHIRO TAKAHASHI, HAEUN YANG, KOJI TSUCHIYA, AYA KONISHI, KINYA UCHIH: "Novel flowcytometry-based approach of malignant cell detection in body fluids using an automated hematology analyzer", PLOS ONE, vol. 13, no. 2, 9 February 2018 (2018-02-09), pages e0190886, XP055743562, DOI: 10.1371/journal.pone.0190886
- BARTEK RAJWA; MURUGESAN VENKATAPATHI; KATHY RAGHEB; PADMAPRIYA P BANADA; DANIEL HIRLEMAN E; TODD LARY; PAUL ROBINSON J: "Automated classification of bacterial particles in flow by multiangle scatter measurement and support vector machine classifier", CYTOMETRY PART A, vol. 73A, no. 4, 28 December 2007 (2007-12-28), pages 369 - 379, XP002648550, DOI: 10.1002/cyto.a.20515
- KIMURA, KONOBU ET AL.: "A novel automated image analysis system using deep convolutional neural networks can assist to differentiate MDS and AA", SICENTIFIC REPORTS, vol. 9, no. 1, 16 September 2019 (2019-09-16), pages 13385, XP055743568, DOI: 10.1038/s41598-019-49942-z
- KIMURA, KONOBU: "Development and validation of a Deep Learning Algorithm for detection of abnormal morphology of WBC", MD ANDERSON CANCER CENTER GLOBAL ACADEMIC PROGRAM (GAP) CONFERENCE, POSTER WS17, Retrieved from the Internet <URL:https://www.juntendo.co.jp/graduate/laboratory/labo/nghlm/news20180515,html>

## Description

### TECHNICAL FIELD

The present specification discloses a cell analysis method, a training method for a deep learning algorithm, a cell analyzer, a training apparatus for a deep learning algorithm, a cell analysis program, and a training program for a deep learning algorithm.

### BACKGROUND ART

Patent Literature 1 discloses a cell analyzer that analyzes the type of a blood cell or the like contained in peripheral blood. In such a cell analyzer, for example, light is applied to each cell in peripheral blood flowing in a flow cell, and signal strengths of scattered light and fluorescence obtained from the cell to which light has been applied are obtained. Peak values of the signal strengths obtained from a plurality of cells are each extracted and plotted on a scattergram. Cluster analysis is performed on the plurality of cells on the scattergram, to identify the type of cells belonging to each cluster.

Patent Literature 2 describes a method for classifying the type of each cell, using an imaging flow cytometer.

### CITATION LIST

### [PATENT LITERATURE]

[PTL 1] Japanese Laid-Open Patent Publication No. S63-180836
[PTL 2] International Publication WO2018/203568
WO 2018/203568 A1 describes that the degree of relation of three or more stages with reference measurement information that is respectively associated with biological measurement information for evaluating a cell is referenced, said reference measurement information being stored in a database in advance; the reference measurement information is retrieved on the basis of the measurement information of a newly measured cell; and the cell is evaluated using the biological measurement information associated with the retrieved reference measurement information.
EP 3 073 265 A1 describes a blood analyzer including a specimen preparation unit, a flow cell, a light source unit, light receivers, and a processing unit. The processing unit is configured to identify and count lymphocytes in the first measurement specimen by using first scattered light information based on the first scattered light and second scattered light information based on the second scattered light, and configured to identify and count blood cells having thereon the predetermined surface antigen in the first measurement specimen by using first fluorescence information based on the first fluorescence.

### SUMMARY OF THE INVENTION

### PROBLEMS TO BE SOLVED BY THE INVENTION

In a case where the type of a cell is to be identified on the basis of a scattergram, when, for example, a cell that usually does not appear in peripheral blood of a healthy individual, such as a blast or a lymphoma cell, is present in a specimen, there are cases where the cell is classified as a normal cell in cluster analysis.

Since the cluster analysis is a statistical analysis technique, when the number of cells plotted on the scattergram is small, the cluster analysis becomes difficult in some cases.

Further, in the method described in Patent Literature 2, in order to perform more accurate determination of the type of each cell, a method of capturing an image of each cell that flows in a flow cell and applying structure illumination is adopted. Therefore, Patent Literature 2 has a problem that a detection system conventionally used for obtaining a scattergram cannot be used.

An object of an embodiment of the present invention is to further improve the accuracy of determination also of different types of cells that appear in the same cluster. Another object of an embodiment of the present invention is to provide a cell type determination method applicable to a measurement apparatus that has conventionally performed measurement on a scattergram.

### SOLUTION TO THE PROBLEMS

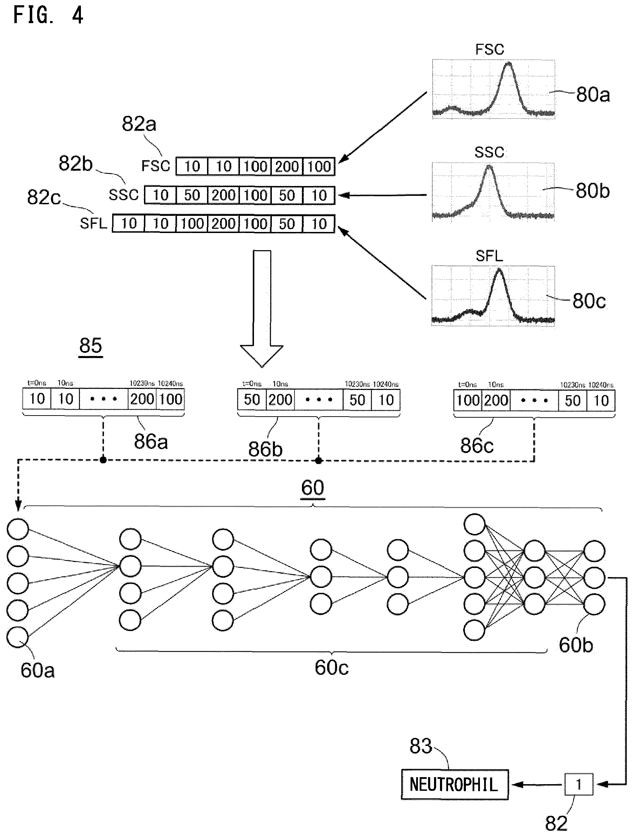

With reference to FIG. 4, a certain embodiment of the present embodiment relates to a cell analysis method for analyzing cells contained in a biological sample, by using a deep learning algorithm (60) having a neural network structure. The cell analysis method includes: causing the cells to flow in a flow path; obtaining a signal strength of a signal regarding each of the individual cells passing through the flow path, and inputting, into the deep learning algorithm (60), numerical data corresponding to the obtained signal strength regarding each of the individual cells; and on the basis of a result outputted from the deep learning algorithm (60), determining, for each cell, a type of the cell for which the signal strength has been obtained. According to the present embodiment, the types of cells that cannot be determined by a conventional cell analyzer can be determined.

In the cell analysis method, preferably, from the individual cells passing through a predetermined position in the flow path, the signal strength is obtained, for each of the cells, at a plurality of time points in a time period while the cell is passing through the predetermined position, and each obtained signal strength is stored in association with information regarding a corresponding time point at which the signal strength has been obtained. According to this embodiment, the types of cells that cannot be determined by a conventional cell analyzer can be determined. Since information regarding the time points at each of which the signal strength has been obtained is obtained, when a plurality of signals have been received from a single cell, data can be synchronized.

In the cell analysis method, preferably, the obtaining of the signal strength at the plurality of time points is started at a time point at which the signal strength of each of the individual cells has reached a predetermined value, and ends after a predetermined time period after the start of the obtaining of the signal strength. According to this embodiment, more accurate determination can be performed. In addition, the volume of data to be obtained can be reduced.

In the cell analysis method, preferably, the signal is a light signal or an electric signal.

More preferably, the light signal is a signal obtained by light being applied to each of the individual cells passing through the flow cell. The predetermined position is a position where the light is applied to each cell in the flow cell (4113, 551). Further preferably, the light is laser light, and the light signal is at least one type selected from a scattered light signal and a fluorescence signal. Still more preferably, the light signal is a side scattered light signal, a forward scattered light signal, and a fluorescence signal. According to this embodiment, the determination accuracy of the types of cells in the flow cytometer can be improved.

In the cell analysis method, the numerical data corresponding to the signal strength inputted to the deep learning algorithm (60) includes information obtained by combining signal strengths of the side scattered light signal, the forward scattered light signal, and the fluorescence signal that have been obtained for each cell at the same time point. According to this embodiment, the determination accuracy by the deep learning algorithm can be further improved.

In the analysis method, when the signal is an electric signal, a measurement part includes a sheath flow electric resistance-type detector. According to this embodiment, the types of cells can be determined on the basis of data measured by a sheath flow electric resistance method.

In the cell analysis method, the deep learning algorithm (60) calculates, for each cell, a probability that the cell for which the signal strength has been obtained belongs to each of a plurality of types of cells associated with an output layer (60b) of the deep learning algorithm (60). Preferably, the deep learning algorithm (60) outputs a label value 82 of a type of a cell that has a highest probability that the cell for which the signal strength has been obtained belongs thereto. According to this embodiment, the determination result can be presented to a user.

In the cell analysis method, on the basis of the label value of the type of the cell that has the highest probability that the cell for which the signal strength has been obtained belongs thereto, the number of cells that belong to each of the plurality of types of cells is counted, and a result of the counting is outputted; or on the basis of the label value of the type of the cell that has the highest probability that the cell for which the signal strength has been obtained belongs thereto, a proportion of cells that belong to each of the plurality of types of cells is calculated, and a result of the calculation is outputted. According to this embodiment, the proportions of the type of cells contained in the biological sample can be obtained.

In the cell analysis method, preferably, the biological sample is a blood sample. More preferably, the type of a cell includes at least one type selected from a group consisting of neutrophil, lymphocyte, monocyte, eosinophil, and basophil. Further preferably, the type of a cell includes at least one type selected from the group consisting of (a) and (b) below. Here, (a) is immature granulocyte; and (b) is at least one type of abnormal cell selected from the group consisting of tumor cell, lymphoblast, plasma cell, atypical lymphocyte, nucleated erythrocyte selected from proerythroblast, basophilic erythroblast, polychromatic erythroblast, orthochromatic erythroblast, promegaloblast, basophilic megaloblast, polychromatic megaloblast, and orthochromatic megaloblast, and megakaryocyte. According to this embodiment, the types of immature granulocytes and abnormal cells contained in a blood sample can be determined.

In the cell analysis method, in a case where the biological sample is a blood sample and the type of cell includes abnormal cell, when there is a cell that has been determined to be an abnormal cell by the deep learning algorithm (60), a processing part (20) may output information indicating that an abnormal cell is contained in the biological sample.

In the cell analysis method, the biological sample may be urine. According to this embodiment, determination can be performed also for cells contained in urine.

A certain embodiment of the present embodiment relates to an analysis method for cells contained in a biological sample. In the cell analysis method, the cells are caused to flow in a flow path; from the individual cells passing through a predetermined position in the flow path, a signal strength regarding each of scattered light and fluorescence is obtained, for each of the cells, at a plurality of time points in a time period while the cell is passing through the predetermined position; and on the basis of a result of recognizing, as a pattern, the obtained signal strengths at the plurality of time points regarding each of the individual cells, a type of the cell is determined for each cell. According to the present embodiment, the types of cells that cannot be determined by a conventional cell analyzer can be determined.

A certain embodiment of the present embodiment relates to a method for training a deep learning algorithm (50) having a neural network structure for analyzing cells in a biological sample. The cells contained in the biological sample are caused to flow in a cell detection flow path in a measurement part capable of detecting cells individually; numerical data corresponding to a signal strength obtained for each of the individual cells passing through the flow path is inputted as first training data to an input layer of the deep learning algorithm; and information of a type of a cell that corresponds to the cell for which the signal strength has been obtained is inputted as second training data to the deep learning algorithm. According to the present embodiment, it is possible to generate a deep learning algorithm for determining the types of individual cells that cannot be determined by a conventional cell analyzer.

A certain embodiment of the present embodiment relates to a cell analyzer (4000, 4000') configured to determine a type of each cell, by using a deep learning algorithm (60) having a neural network structure. The cell analyzer (4000, 4000') includes a processing part (20). The processing part (20) is configured to: obtain, when cells contained in a biological sample and caused to pass through a cell detection flow path in a measurement part capable of detecting cells individually, a signal strength regarding each of the individual cells; input, to the deep learning algorithm (60), numerical data corresponding to the obtained signal strength regarding each of the individual cells; and on the basis of a result outputted from the deep learning algorithm, determine, for each cell, a type of the cell for which the signal strength has been obtained. According to the present embodiment, the types of cells that cannot be determined by a conventional cell analyzer can be determined.

Further, the cell analyzer (4000, 4000') includes a measurement part (400) capable of detecting cells individually and configured to obtain, when the cells contained in the biological sample and caused to flow in the cell detection flow path of the measurement part pass through the flow path, a signal strength regarding each of the individual cells. According to the present embodiment, due to the cell analyzer including the measurement part, the types of cells that cannot be determined by a conventional cell analyzer can be determined.

A certain embodiment of the present embodiment relates to a training apparatus (100) for training a deep learning algorithm (50) having a neural network structure for analyzing cells in a biological sample. The training apparatus includes a processing part (100). The processing part (10) is configured to: cause the cells contained in the biological sample to flow in a cell detection flow path in a measurement part capable of detecting cells individually, and input, as first training data to an input layer of the deep learning algorithm, numerical data corresponding to a signal strength obtained for each of the individual cells passing through the flow path; and input, as second training data to the deep learning algorithm, information of a type of a cell that corresponds to the cell for which the signal strength has been obtained. According to the present embodiment, it is possible to generate a deep learning algorithm for determining the types of cells that cannot be determined by a conventional cell analyzer.

A certain embodiment of the present embodiment relates to a computer program for analyzing cells contained in a biological sample, by using a deep learning algorithm (60) having a neural network structure. The computer program is configured to cause a processing part (20) to execute a process including: a step of causing the cells contained in the biological sample to flow in a cell detection flow path in a measurement part capable of detecting cells individually, and obtaining a signal strength regarding each of the individual cells passing through the flow path; a step of inputting, to the deep learning algorithm, numerical data corresponding to the obtained signal strength regarding each of the individual cells; and a step of, on the basis of a result outputted from the deep learning algorithm, determining, for each cell, a type of the cell for which the signal strength has been obtained. According to the present embodiment, due to the cell analyzer including the measurement part, the types of cells that cannot be determined by a conventional cell analyzer can be determined.

A certain embodiment of the present embodiment relates to a computer program for training a deep learning algorithm (50) having a neural network structure for analyzing cells in a biological sample. The computer program is configured to cause a processing part (10) to execute a process including: a step of causing the cells contained in the biological sample to flow in a cell detection flow path in a measurement part capable of detecting cells individually, and inputting, as first training data to an input layer of the deep learning algorithm, numerical data corresponding to a signal strength obtained for each of the individual cells passing through the flow path; and a step of inputting, as second training data to the deep learning algorithm, information of a type of a cell that corresponds to the cell for which the signal strength has been obtained. According to the present embodiment, it is possible to generate a deep learning algorithm for determining the types of cells that cannot be determined by a conventional cell analyzer.

### ADVANTAGEOUS EFFECTS OF THE INVENTION

The types of cells that cannot be determined by a conventional cell analysis method can be determined. Therefore, the determination accuracy for cells can be improved.

### BRIEF DESCRIPTION OF THE DRAWINGS

[FIG. 1] (a) shows an example of a scattergram of blood of a healthy individual. (b) shows an example of a scattergram of unhealthy blood. (c) shows a display example in a conventional scattergram. (d) shows an example of waveform data. (f) shows a schematic diagram of a deep learning algorithm. (g) shows a cell determination example.
[FIG. 2] FIG. 2 shows an example of a generation method for training data.
[FIG. 3] FIG. 3 shows an example of a label value.
[FIG. 4] FIG. 4 shows an example of a generation method for analysis data.
[FIG. 5A] FIG. 5A shows an example of the appearance of a cell analyzer.
[FIG. 5B] FIG. 5B shows an example of the appearance of a cell analyzer.
[FIG. 6] FIG. 6 shows a block diagram of a measurement unit.
[FIG. 7] FIG. 7 shows a schematic example of an optical system of a flow cytometer.
[FIG. 8] FIG. 8 shows a schematic example of a sample preparation part of the measurement unit.
[FIG. 9A] FIG. 9A shows a schematic example of a red blood cell/platelet detector.
[FIG. 9B] FIG. 9B shows a histogram of cells detected by a sheath flow electric resistance method.
[FIG. 10] FIG. 10 shows a block diagram of a measurement unit.
[FIG. 11] FIG. 11 shows a schematic example of an optical system of a flow cytometer.
[FIG. 12] FIG. 12 shows a schematic example of a sample preparation part of the measurement unit.
[FIG. 13] FIG. 13 shows a schematic example of a waveform data analysis system.
[FIG. 14] FIG. 14 shows a block diagram of a vendor-side apparatus.
[FIG. 15] FIG. 15 shows a block diagram of a user-side apparatus.
[FIG. 16] FIG. 16 shows an example of a function block diagram of a vendor-side apparatus.
[FIG. 17] FIG. 17 shows an example of a flow chart of operation performed by a processing part for generating training data.
[FIG. 18A] FIG. 18A is a schematic diagram for describing a neural network and the schematic diagram shows the outline of the neural network.
[FIG. 18B] FIG. 18B is a schematic diagram for describing a neural network and the schematic diagram shows calculation at each node.
[FIG. 18C] FIG. 18C is a schematic diagram for describing a neural network and the schematic diagram shows calculation between nodes.
[FIG. 19] FIG. 19 shows an example of a function block diagram of a user-side apparatus.
[FIG. 20] FIG. 20 shows an example of a flow chart of operation performed by a processing part for generating analysis data.
[FIG. 21] FIG. 21 shows a schematic example of a waveform data analysis system.
[FIG. 22] FIG. 22 shows a function block diagram of the waveform data analysis system.
[FIG. 23] FIG. 23 shows a schematic example of a waveform data analysis system.
[FIG. 24] FIG. 24 shows a function block diagram of the waveform data analysis system.
[FIG. 25] FIG. 25 shows an example of output data.
[FIG. 26] FIG. 26 shows a mix matrix of a determination result by a reference method and a determination result obtained by using the deep learning algorithm.
[FIG. 27A] FIG. 27A shows an ROC curve of neutrophil.
[FIG. 27B] FIG. 27B shows an ROC curve of lymphocyte.
[FIG. 27C] FIG. 27C shows an ROC curve of monocyte.
[FIG. 28A] FIG. 28A shows an ROC curve of eosinophil.
[FIG. 28B] FIG. 28B shows an ROC curve of basophil.
[FIG. 28C] FIG. 28C shows an ROC curve of control blood (CONT).

### BEST MODE FOR CARRYING OUT THE INVENTION

Hereinafter, the outline and embodiments of the present invention will be described in detail with reference to the attached drawings. In the description below and the drawings, the same reference characters represent the same or similar components. Thus, description of the same or similar components is not repeated.

### [1. Cell analysis method]

The present embodiment relates to a cell analysis method for analyzing cells contained in a biological sample. In the analysis method, numerical data corresponding to a signal strength regarding each of individual cells is inputted to a deep learning algorithm that has a neural network structure. Then, on the basis of the result outputted from the deep learning algorithm, the type of the cell for which the signal strength has been obtained is determined for each cell.

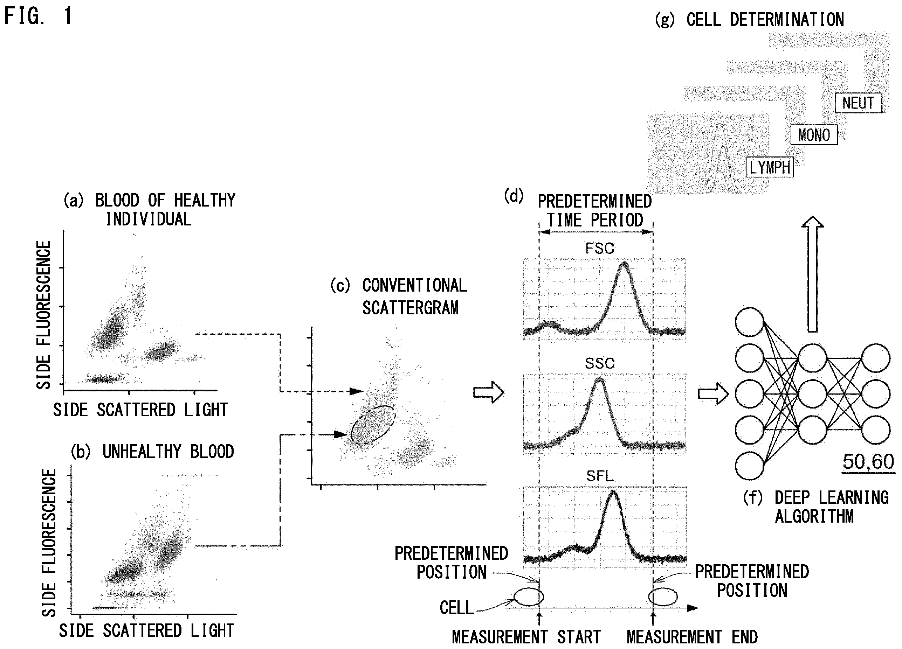

With reference to FIG. 1, an example of the outline of the present embodiment is described. In FIG. 1, (a) shows a scattergram of results obtained by measuring, with a flow cytometer, signal strengths of fluorescence and scattered light of individual cells contained in a biological sample, using healthy blood as a biological sample. The horizontal axis represents the signal strength of side scattered light and the vertical axis represents the signal strength of side fluorescence. Similar to (a), (b) is a scattergram of results obtained by measuring, with a flow cytometer, signal strengths of side fluorescence and side scattered light of individual cells contained in a biological sample, using unhealthy blood as a biological sample. Each of the diagrams shown in (a) and (b) is used in conventional white blood cell classification using a flow cytometer. However, in general, when unhealthy blood cells are contained in blood, unhealthy blood cells and healthy blood cells are mixed in the blood. Therefore, as shown in (c), there are cases where dots of healthy blood cells and dots of unhealthy blood cells overlap each other.

The present embodiment is focused on data indicating the signal strength that is derived from each of individual cells and that is obtained when creating a scattergram. In (d) of FIG. 1, FSC represents data indicating the signal strength of forward scattered light, SSC represents waveform data of side scattered light, and SFL represents data indicating the signal strength of side fluorescence. Here, (d) of FIG. 1 shows waveforms that are rendered for convenience. However, in the present embodiment, the data indicated in the form of a waveform is intended to mean a data group whose elements are values each indicating the time of obtainment of a signal strength, and values each indicating the signal strength at that time point, and is not intended to mean the shape itself of the rendered waveform. The data group means sequence data or matrix data. In (d) of FIG. 1, obtainment of a signal strength is started when individual cells pass through a predetermined position, and after a predetermined time period, measurement is started.

In the present embodiment, a deep learning algorithm 50, 60 shown in (f) of FIG. 1 is caused to learn waveform data of each type of cell, and on the basis of the result outputted from the deep learning algorithm having learned, a determination result ((g) of FIG. 1) of the types of individual cells contained in a biological sample is produced. Hereinafter, each of individual cells in a biological sample subjected to analysis for the purpose of determining the type of cell will also be referred to as an "analysis target cell". In other words, a biological sample can contain a plurality of analysis target cells. A plurality of cells can include a plurality of types of analysis target cells.

An example of a biological sample is a biological sample collected from a subject. Examples of the biological sample can include blood such as peripheral blood, venous blood, or arterial blood, urine, and a body fluid other than blood and urine. Examples of the body fluid other than blood and urine can include bone marrow, ascites, pleural effusion, spinal fluid, and the like. Hereinafter, the body fluid other than blood and urine may be simply referred to as a "body fluid". The blood sample may be any blood sample that is in a state where the number of cells can be counted and the types of cells can be determined. Preferably, blood is peripheral blood. Examples of blood include peripheral blood collected using an anticoagulant agent such as ethylenediamine tetraacetate (sodium salt or potassium salt), heparin sodium, or the like. Peripheral blood may be collected from an artery or may be collected from a vein.

The types of cells to be determined in the present embodiment are those according to the types of cells based on morphological classification, and are different depending on the kind of the biological sample. When the biological sample is blood and the blood is collected from a healthy individual, the types of cells to be determined in the present embodiment include red blood cell, nucleated cell such as white blood cell, platelet, and the like. Nucleated cells include neutrophils, lymphocytes, monocytes, eosinophils, and basophils. Neutrophils include segmented neutrophils and band neutrophils. Meanwhile, when blood is collected from an unhealthy individual, nucleated cells may include at least one type selected from the group consisting of immature granulocyte and abnormal cell. Such cells are also included in the types of cells to be determined in the present embodiment. Immature granulocytes can include cells such as metamyelocytes, bone marrow cells, promyelocytes, and myeloblasts.

The nucleated cells may include abnormal cells that are not contained in peripheral blood of a healthy individual, in addition to normal cells. Examples of abnormal cells are cells that appear when a person has a certain disease, and such abnormal cells are tumor cells, for example. In a case of the hematopoietic system, the certain disease can be a disease selected from the group consisting of: myelodysplastic syndrome; leukemia such as acute myeloblastic leukemia, acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphoblastic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma.

Further, abnormal cells can include cells that are not usually observed in peripheral blood of a healthy individual, such as: lymphoblasts; plasma cells; atypical lymphocytes; reactive lymphocytes; erythroblasts, which are nucleated erythrocytes, such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts; megakaryocytes including micromegakaryocytes; and the like.

When the biological sample is urine, the types of cells to be determined in the present embodiment can include red blood cells, white blood cells, epithelial cells such as those of transitional epithelium, squamous epithelium, and the like. Examples of abnormal cells include bacteria, fungi such as filamentous fungi and yeast, tumor cells, and the like.

When the biological sample is a body fluid that usually does not contain blood components, such as ascites, pleural effusion, or spinal fluid, the types of cells can include red blood cell, white blood cell, and large cell. The "large cell" here means a cell that is separated from an inner membrane of a body cavity or a peritoneum of a viscus, and that is larger than white blood cells. Specifically, mesothelial cells, histiocytes, tumor cells, and the like correspond to the "large cell".

When the biological sample is bone marrow, the types of cells to be determined in the present embodiment can include, as normal cells, mature blood cells and immature hematopoietic cells. Mature blood cells include red blood cells, nucleated cells such as white blood cells, platelets, and the like. Nucleated cells such as white blood cells include neutrophils, lymphocytes, plasma cells, monocytes, eosinophils, and basophils. Neutrophils include segmented neutrophils and band neutrophils. Immature hematopoietic cells include hematopoietic stem cells, immature granulocytic cells, immature lymphoid cells, immature monocytic cells, immature erythroid cells, megakaryocytic cells, mesenchymal cells, and the like. Immature granulocytes can include cells such as metamyelocytes, bone marrow cells, promyelocytes, and myeloblasts. Immature lymphoid cells include lymphoblasts and the like. Immature monocytic cells include monoblasts and the like. Immature erythroid cells include nucleated erythrocytes such as proerythroblasts, basophilic erythroblasts, polychromatic erythroblasts, orthochromatic erythroblasts, promegaloblasts, basophilic megaloblasts, polychromatic megaloblasts, and orthochromatic megaloblasts. Megakaryocytic cells include megakaryoblasts, and the like.

Examples of abnormal cells that can be included in bone marrow include hematopoietic tumor cells of a disease selected from the group consisting of: myelodysplastic syndrome; leukemia such as acute myeloblastic leukemia, acute myeloblastic leukemia, acute promyelocytic leukemia, acute myelomonocytic leukemia, acute monocytic leukemia, erythroleukemia, acute megakaryoblastic leukemia, acute myeloid leukemia, acute lymphoblastic leukemia, lymphoblastic leukemia, chronic myelogenous leukemia, or chronic lymphocytic leukemia; malignant lymphoma such as Hodgkin's lymphoma or non-Hodgkin's lymphoma; and multiple myeloma, which have been described above, and metastasized tumor cells of a malignant tumor developed in an organ other than bone marrow.

FIG. 1 shows an example of using, as a signal, a light signal (forward scattered light signal, side scattered light signal, side fluorescence signal). However, the signal may be an electric signal, for example. The light signal is a signal of light emitted from a cell when light is applied to the cell. The light signal can include at least one type selected from a scattered light signal and a fluorescence signal. In the present specification, light can be applied so as to be orthogonal to the flow of cells in a flow path, for example. "Forward" means the advancing direction of light emitted from a light source. When the angle of application light is defined as 0 degrees, "forward" can include a forward low angle at which the light reception angle is about 0 to 5 degrees, and/or a forward high angle at which the light reception angle is about 5 to 20 degrees. "Side" is not limited as long as the "side" does not overlap "forward". When the angle of application light is defined as 0 degrees, "side" can include a light reception angle being about 25 degrees to 155 degrees, preferably about 45 degrees to 135 degrees, and more preferably about 90 degrees. In the present embodiment, irrespective of the kind of the signal, a data group (sequence data or matrix data, preferably one-dimensional sequence data) whose elements are values each indicating the time of obtainment of a signal strength, and values each indicating the signal strength at that time point may be collectively referred to as waveform data.

In the cell analysis method of the present embodiment, the determination method of the type of cell is not limited to a method that uses a deep learning algorithm. From individual cells passing through a predetermined position in a flow path, a signal strength is obtained, for each of the cells, at a plurality of time points in a time period while the cell is passing through the predetermined position, and on the basis of a result obtained by recognizing, as a pattern, the obtained signal strengths at the plurality of time points regarding the individual cells, the types of cells may be determined. The pattern may be recognized as a numerical pattern of signal strengths at a plurality of time points, or may be recognized as a shape pattern obtained when signal strengths at a plurality of time points are plotted on a graph. When the pattern is recognized as a numerical pattern, if a numerical pattern of an analysis target cell and a numerical pattern for which the type of cell is already known are compared with each other, the type of cell can be determined. For the comparison between the numerical pattern of an analysis target cell and a control numerical pattern, Spearman rank correlation, z-score, or the like can be used, for example. When the pattern of the graph shape of an analysis target cell and the pattern of a graph shape for which the type of cell is already known are compared with each other, the type of cell can be determined. For the comparison between the pattern of the graph shape of an analysis target cell and the pattern of the graph shape for which the type of cell is already known, geometric shape pattern matching may be used, or a feature descriptor represented by SIFT Descriptor may be used, for example.

### <Outline of cell analysis method>

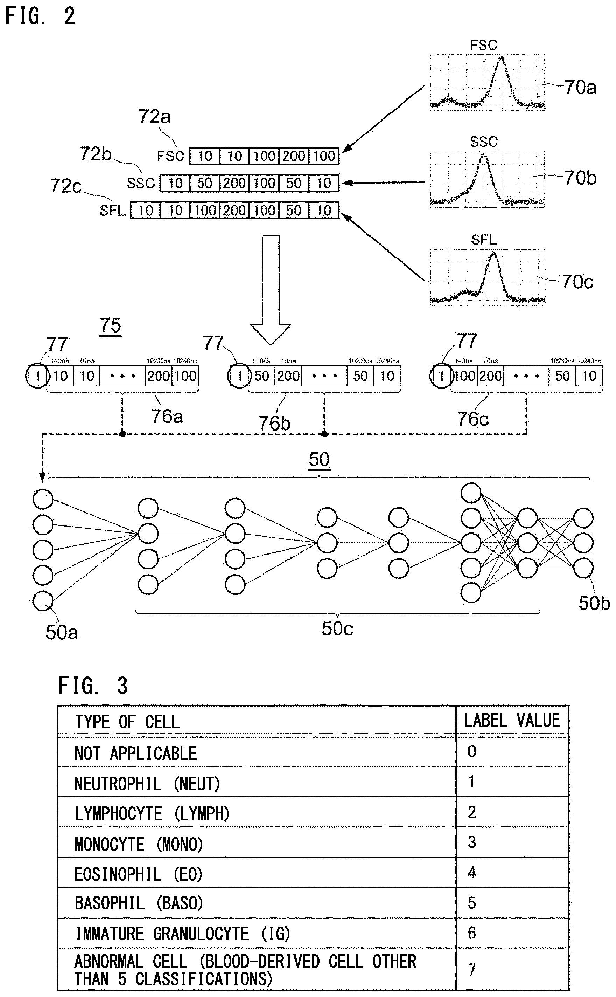

Next, with reference to the examples shown in FIG. 2 to FIG. 4, a generation method for training data 75 and an analysis method for waveform data are described.

### <Generation of training data>

The example shown in FIG. 2 is an example of a generation method for training waveform data to be used in order to train a deep learning algorithm for determining the types of white blood cells, immature granulocytes, and abnormal cells. Waveform data 70a of forward scattered light, waveform data 70b of side scattered light, and waveform data 70c of side fluorescence are associated with a training target cell. The training waveform data 70a, 70b, 70c obtained from the training target cell may be waveform data obtained by measuring, through flow cytometry, a cell for which the kind of cell based on morphological classification is known. Alternatively, waveform data of a cell for which the type of cell has already been determined from a scattergram of a healthy individual, may be used. As the waveform data for which the type of cell, of a healthy individual, has been determined, a pool of waveform data of cells obtained from a plurality of persons may be used. A specimen for obtaining the training waveform data 70a, 70b, 70c is preferably a sample that contains the same type of cell as the training target cell, and that is treated by a specimen treatment method similar to that for a specimen that contains the training target cell. The training waveform data 70a, 70b, 70c is preferably obtained under a condition similar to the condition for obtaining the analysis target cell. The training waveform data 70a, 70b, 70c can be obtained in advance for each cell by, for example, a known flow cytometry or sheath flow electric resistance method. Here, when the training target cell is a red blood cell or a platelet, the training data is waveform data obtained by a sheath flow electric resistance method, and the waveform data may be of a single type obtained from an electric signal strength.

In the example shown in FIG. 2, training waveform data 70a, 70b, 70c obtained through flow cytometry by using Sysmex XN-1000 is used. The training waveform data 70a, 70b, 70c is an example in which, for example, during a time period from the start, upon forward scattered light reaching a predetermined threshold, of obtainment of the signal strength of forward scattered light, the signal strength of side scattered light, and the signal strength of side fluorescence, until the end of the obtainment after a predetermined time period, each piece of waveform data is obtained for a single training target cell at a plurality of time points at a certain interval. For example, obtainment of waveform data at a plurality of time points at a certain interval is performed at 1024 points at a 10 nanosecond interval, at 128 points at an 80 nanosecond interval, 64 points at a 160 nanosecond interval, or the like. As for each piece of waveform data, cells contained in a biological sample are caused to flow in a cell detection flow path in a measurement part that is capable of detecting cells individually and that is provided in a flow cytometer, a sheath flow electric resistance-type measurement apparatus, or the like, and each piece of waveform data is obtained for each of the individual cells passing through the flow path. Specifically, at a plurality of time points in a time period while a single training target cell is passing through a predetermined position in the flow path, a data group whose elements are values each indicating the time of obtainment of a signal strength and values each indicating the signal strength at that time point, is obtained for each signal, and is used as the training waveform data 70a, 70b, 70c. Information of each time point is not limited as long as the information can be stored such that processing parts 10, 20 described later can determine how much time has elapsed since the start of obtainment of the signal strength. For example, the information of the time point may be a time period from the measurement start, or may be information that indicates what number the point is. Each signal strength is preferably stored in a storage 13, 23 or a memory 12, 22 described later, together with the information of the time point at which the signal strength has been obtained.

When the respective pieces of the training waveform data 70a, 70b, 70c in FIG. 2 are indicated in the form of raw data values, sequence data 72a of forward scattered light, sequence data 72b of side scattered light, and sequence data 72c of side fluorescence are obtained, for example. With respect to the sequence data 72a, 72b, 72c, the time points of obtainment of the signal strengths are synchronized for each training target cell, and sequence data 76a of forward scattered light, sequence data 76b of side scattered light, and sequence data 76c of side fluorescence are obtained. That is, the second numerical value from the left in 76a is 10 as the signal strength at a time t=0 at which measurement was started. Similarly, the second numerical values from the left in 76b and 76c are 50 and 100, respectively, as the signal strengths at the time t=0 at which measurement was started. Cells that are adjacent to each other in each of 76a, 76b, and 76c store signal strengths at a 10 nanosecond interval. The pieces of the sequence data 76a, 76b, 76c are each combined with a label value 77 indicating the type of the training target cell and are combined such that three signal strengths (a signal strength of forward scattered light, a signal strength of side scattered light, and a signal strength of side fluorescence) at the same time point form one set, and then, the resultant set is inputted as the training data 75 to the deep learning algorithm 50. For example, when the training target cell is a neutrophil, the sequence data 76a, 76b, 76c is provided with "1" as a label value 77 representing a neutrophil, and the training data 75 is generated. FIG. 3 shows an example of the label value 77. Since the training data 75 is generated for each type of cell, a different label value 77 is provided in accordance with the kind of cell. Here, synchronization of the time points of obtainment of signal strengths means matching the measurement points such that, for example, the time periods from the measurement start are aligned, at the same time point, as a combination with respect to the sequence data 72a of forward scattered light, the sequence data 72b of side scattered light, and the sequence data 72c of side fluorescence. In other words, the sequence data 72a of forward scattered light, the sequence data 72b of side scattered light, and the sequence data 72c of side fluorescence are adjusted so as to have signal strengths obtained at the same time point from a single cell passing through the flow cell. The time of measurement start may be a time point at which the signal strength of forward scattered light has exceeded a predetermined threshold, for example. However, a threshold for a signal strength of another scattered light or fluorescence may be used. Alternatively, a threshold may be set for each piece of sequence data.

For the sequence data 76a, 76b, 76c, the obtained signal strength values may be directly used, but processing such as noise removal, baseline correction, and normalization may be performed as necessary. In the present specification, "numerical data corresponding to a signal strength" can include an obtained signal strength value itself, and a value that has been subjected to noise removal, baseline correction, normalization, and the like as necessary.

### <Outline of deep learning>

With reference to FIG. 2 used as an example, the outline of training of a neural network is described. The neural network 50 is preferably a convolution neural network. The number of nodes in an input layer 50a in the neural network 50 corresponds to the number of sequences included in the waveform data of the training data 75 to be inputted. In the training data 75, the pieces of the sequence data 76a, 76b, 76c are combined such that the time points of obtainment of the signal strengths are aligned at the same time point, and the training data 75 is inputted as first training data to the input layer 50a of the neural network 50. The label value 77 of each piece of waveform data of the training data 75 is inputted as second training data to an output layer 50b of the neural network, to train the neural network 50. The reference character 50c in FIG. 2 represents a middle layer.

### <Analysis method for waveform data>

FIG. 4 shows an example of a method for analyzing waveform data of a cell as an analysis target. In the analysis method for waveform data, analysis data 85 is generated from waveform data 80a of forward scattered light, waveform data 80b of side scattered light, and waveform data 80c of side fluorescence, which have been obtained from an analysis target cell. The analysis waveform data 80a, 80b, 80c can be obtained by using known flow cytometry, for example. In the example shown in FIG. 4, similar to the training waveform data 70a, 70b, 70c, the analysis waveform data 80a, 80b, 80c is obtained by using Sysmex XN-1000. When the respective pieces of the analysis waveform data 80a, 80b, 80c are indicated in the form of raw data values, sequence data 82a of forward scattered light, waveform data 82b of side scattered light, and waveform data 82c of side fluorescence are obtained, for example.

Preferably, at least the obtainment condition and the condition for generating, from each piece of waveform data or the like, data to be inputted to the neural network are the same between generation of the analysis data 85 and generation of the training data 75. With respect to the sequence data 82a, 82b, 82c, for each training target cell, the time points of obtainment of the signal strengths are synchronized, and sequence data 86a (forward scattered light), sequence data 86b (side scattered light), and sequence data 86c (side fluorescence) are obtained. The sequence data 86a, 86b, 86c are combined such that three signal strengths (a signal strength of forward scattered light, a signal strength of side scattered light, and a signal strength of side fluorescence) at the same time point form one set, and is inputted as the analysis data 85 to the deep learning algorithm 60.

When the analysis data 85 has been inputted to an input layer 60a of the neural network 60 serving as a trained deep learning algorithm 60, a probability that the analysis target cell from which the analysis data 85 has been obtained belongs to each of types of cells inputted as training data is outputted from an output layer 60b. The reference character 60c in FIG. 4 represents a middle layer. Further, it may be determined that the analysis target cell from which the analysis data 85 has been obtained belongs to a classification that corresponds to the highest value among the probabilities, and a label value 82 or the like associated with the type of cell may be outputted. An analysis result 83 to be outputted regarding the cell may be the label value itself, or may be data obtained by replacing the label value with information (e.g., a term) that indicates the type of cell. In the example in FIG. 4, on the basis of the analysis data 85, the deep learning algorithm 60 outputs a label value "1", which has the highest probability that the analysis target cell from which the analysis data 85 has been obtain belongs thereto. In addition, character data "neutrophil" corresponding to this label value is outputted as the analysis result 83 regarding the cell. The output of the label value may be performed by the deep learning algorithm 60, but another computer program may output a most preferable label value on the basis of the probabilities calculated by the deep learning algorithm 60.

### [2. Cell analyzer and measurement of biological sample in the cell analyzer]

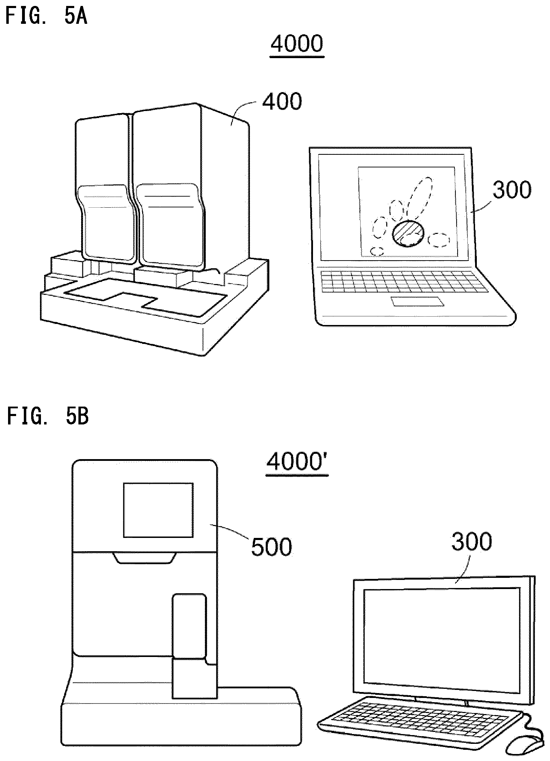

Waveform data according to the present embodiment can be obtained in a first cell analyzer 4000 or a second cell analyzer 4000'. FIG. 5A shows the appearance of the cell analyzer 4000. FIG. 5B shows the appearance of the cell analyzer 4000'. In FIG. 5A, the cell analyzer 4000 includes: a measurement unit (also referred to as a measurement part) 400; and a processing unit 300 for controlling settings of the measurement condition for a sample and measurement in the measurement unit 400. In FIG. 5B, the cell analyzer 4000' includes: a measurement unit (also referred to as a measurement part) 500; and a processing unit 300 for controlling settings of the measurement condition for a sample and measurement in the measurement unit 500. The measurement unit 400, 500 and the processing unit 300 can be communicably connected to each other in a wired or wireless manner. A configuration example of the measurement unit 400, 500 is shown below, but implementation of the present embodiment should not be construed to be limited to the example below. The processing unit 300 may be used in common by a vendor apparatus 100 or a user apparatus 200 described later. The block diagram of the processing unit 300 is the same as that of the vendor apparatus 100 or the user apparatus 200.

### <First cell analyzer and preparation of measurement sample>

### (Configuration of first measurement unit)

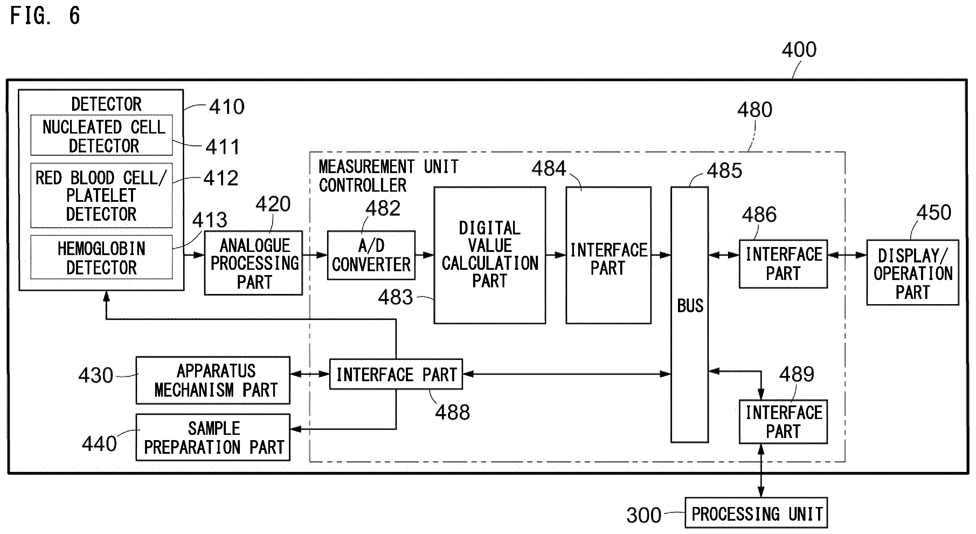

With reference to FIG. 6 to FIG. 8, a configuration example (measurement unit 400) when the first measurement unit 400 is a flow cytometer for detecting nucleated cells in a blood sample is described.

FIG. 6 shows an example of a block diagram of the measurement unit 400. As shown in FIG. 6, the measurement unit 400 includes: a detector 410 for detecting blood cells; an analogue processing part 420 for an output from the detector 410; a measurement unit controller 480; a display/operation part 450; a sample preparation part 440; and an apparatus mechanism part 430. The analogue processing part 420 performs processing including noise removal on an electric signal as an analogue signal inputted from the detector, and outputs the processed result as an electric signal to an A/D converter 482.

The detector 410 includes: a nucleated cell detector 411 which detects nucleated cells such as white blood cells at least; a red blood cell/platelet detector 412 which measures the number of red blood cells and the number of platelets; and a hemoglobin detector 413 which measures the amount of hemoglobin in blood as necessary. The nucleated cell detector 411 is implemented as an optical detector, and more specifically, includes a component for performing detection by flow cytometry.

As shown in FIG. 6, the measurement unit controller 480 includes: the A/D converter 482; a digital value calculation part 483; and an interface part 489 connected to the processing unit 300. Further, the measurement unit controller 480 includes: an interface part 486 for the display/operation part 450; and an interface part 488 for the apparatus mechanism part 430.

The digital value calculation part 483 is connected to the interface part 489 via an interface part 484 and a bus 485. The interface part 489 is connected to the display/operation part 450 via the bus 485 and the interface part 486, and is connected to the detector 410, the apparatus mechanism part 430, and a sample preparation part 440 via the bus 485 and the interface part 488.

The A/D converter 482 converts a reception light signal, which is an analogue signal outputted from the analogue processing part 420, into a digital signal, and outputs the digital signal to the digital value calculation part 483. The digital value calculation part 483 performs predetermined arithmetic processing on the digital signal outputted from the A/D converter 482. Examples of the predetermined arithmetic processing include, but not limited to: a process in which, during a time period from the start, upon forward scattered light reaching a predetermined threshold, of obtainment of the signal strength of forward scattered light, the signal strength of side scattered light, and the signal strength of side fluorescence, until the end of the obtainment after a predetermined time period, each piece of waveform data is obtained for a single training target cell at a plurality of time points at a certain interval; a process of extracting a peak value of the waveform data; and the like. Then, the digital value calculation part 483 outputs the calculation result (measurement result) to the processing unit 300 via the interface part 484, the bus 485, and the interface part 489.

The processing unit 300 is connected to the digital value calculation part 483 via the interface part 484, the bus 485, and the interface part 489, and the processing unit 300 can receive the calculation result outputted from the digital value calculation part 483. In addition, the processing unit 300 performs control of the apparatus mechanism part 430 including a sampler (not shown) that automatically supplies sample containers, a fluid system for preparation/measurement of a sample, and the like, and performs other controls.

The nucleated cell detector 411 causes a measurement sample containing cells to flow in a cell detection flow path, applies light to each cell flowing in the cell detection flow path, and measures scattered light and fluorescence generated from the cell. The red blood cell/platelet detector 412 causes a measurement sample containing cells to flow in a cell detection flow path, measures electric resistance of each cell flowing in the cell detection flow path, and detects the volume of the cell.

In the present embodiment, the measurement unit 400 preferably includes a flow cytometer and/or a sheath flow electric resistance-type detector. In FIG. 6, the nucleated cell detector 411 can be a flow cytometer. In FIG. 6, the red blood cell/platelet detector 412 can be a sheath flow electric resistance-type detector. Here, nucleated cells may be measured by the red blood cell/platelet detector 412, and red blood cells and platelets may be measured by the nucleated cell detector 411.

### Flow cytometer

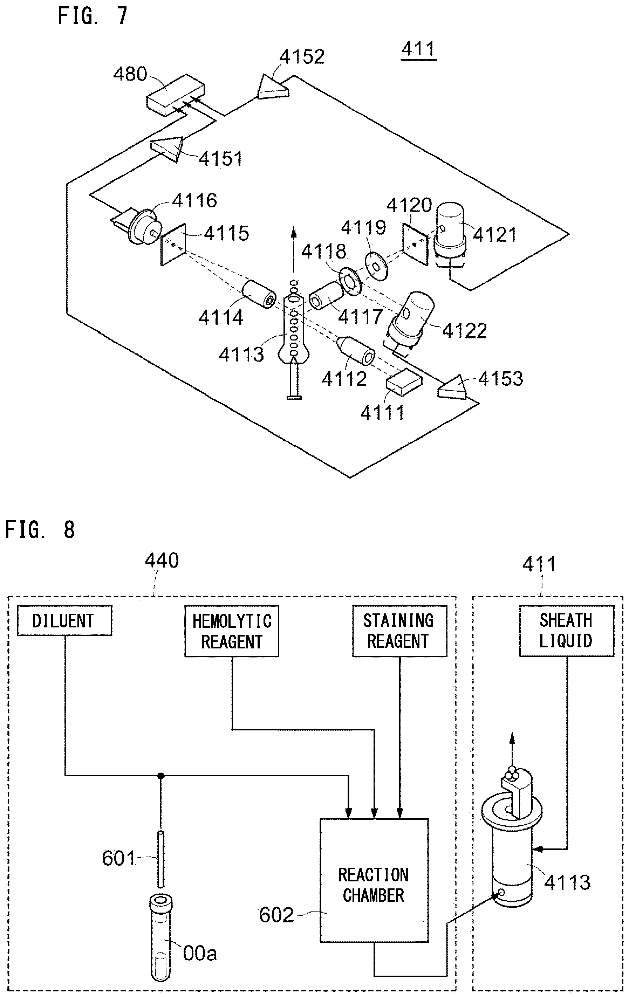

As shown in FIG. 7, in measurement performed by a flow cytometer, when each cell contained in a measurement sample passes through a flow cell (sheath flow cell) 4113 provided in the flow cytometer, a light source 4111 applies light to the flow cell 4113, and scattered light and fluorescence emitted from the cell in the flow cell 4113 due to this light are detected.

In the present embodiment, scattered light may be any scattered light that can be measured by a flow cytometer that is distributed in general. Examples of scattered light include forward scattered light (e.g., light reception angle: about 0 to 20 degrees), and side scattered light (light reception angle: about 90 degrees). It is known that side scattered light reflects internal information of a cell, such as a nucleus or granules of the cell, and forward scattered light reflects information of the size of the cell. In the present embodiment, forward scattered light intensity and side scattered light intensity are preferably measured as scattered light intensity.

Fluorescence is light that is emitted from a fluorescent dye bound to a nucleic acid or the like in a cell when excitation light having an appropriate wavelength is applied to the fluorescent dye. The excitation light wavelength and the reception light wavelength depend on the kind of the fluorescent dye that is used.

FIG. 7 shows a configuration example of an optical system of the nucleated cell detector 411. In FIG. 7, light emitted from a laser diode serving as the light source 4111 is applied via a light application lens system 4112 to each cell passing through the flow cell 4113.

In the present embodiment, the light source 4111 of the flow cytometer is not limited in particular, and a light source 201 that has a wavelength suitable for excitation of the fluorescent dye is selected. As such a light source 201, a semiconductor laser including a red semiconductor laser and/or a blue semiconductor laser, a gas laser such as an argon laser or a helium-neon laser, a mercury arc lamp, or the like is used, for example. In particular, a semiconductor laser is suitable because the semiconductor laser is very inexpensive when compared with a gas laser.

As shown in FIG. 7, forward scattered light emitted from the particle passing through the flow cell 4113 is received by a forward scattered light receiving element 4116 via a condenser lens 4114 and a pinhole part 4115. The forward scattered light receiving element 4116 can be a photodiode or the like. Side scattered light is received by a side scattered light receiving element 4121 via a condenser lens 4117, a dichroic mirror 4118, a bandpass filter 4119, and a pinhole part 4120. The side scattered light receiving element 4121 can be a photodiode, a photomultiplier, or the like. Side fluorescence is received by a side fluorescence receiving element 4122 via the condenser lens 4117 and the dichroic mirror 4118. The side fluorescence receiving element 4122 can be an avalanche photodiode, a photomultiplier, or the like.

Reception light signals outputted from the respective light receiving elements 4116, 4121, and 4122 are subjected to analogue processing such as amplification/waveform processing by the analogue processing part 420 shown in FIG. 6 and having amplifiers 4151, 4152, and 4153, and then, are sent to the measurement unit controller 480.

With reference back to FIG. 6, the measurement part 400 may include the sample preparation part 440 which prepares a measurement sample. The sample preparation part 440 is controlled by a measurement unit information processing part 481 via the interface part 488 and the bus 485. FIG. 8 shows how, in the sample preparation part 440 provided in the measurement part 400, a blood sample, a staining reagent, and a hemolytic reagent are mixed to prepare a measurement sample, and the obtained measurement sample is measured by the nucleated cell detector.

In FIG. 8, a blood sample in a sample container 00a is suctioned by a suction pipette 601. The blood sample quantified by the suction pipette 601 is mixed with a predetermined amount of a diluent, and the resultant mixture is transferred to a reaction chamber 602. A predetermined amount of the hemolytic reagent is added to the reaction chamber 602. A predetermined amount of the staining reagent is supplied to the reaction chamber 602, to be mixed with the above mixture. The mixture of the blood sample, the staining reagent, and the hemolytic reagent is reacted in the reaction chamber 602 for a predetermined time period, whereby red blood cells in the blood sample are hemolyzed, and a measurement sample in which nucleated cells are stained by a fluorescent dye is obtained.

The obtained measurement sample is sent to the flow cell 4113 in the nucleated cell detector 411, together with a sheath liquid (e.g., CELLPACK (II) manufactured by Sysmex Corporation), to be measured by flow cytometry in the nucleated cell detector 411.

### Sheath flow-type electric resistance detector

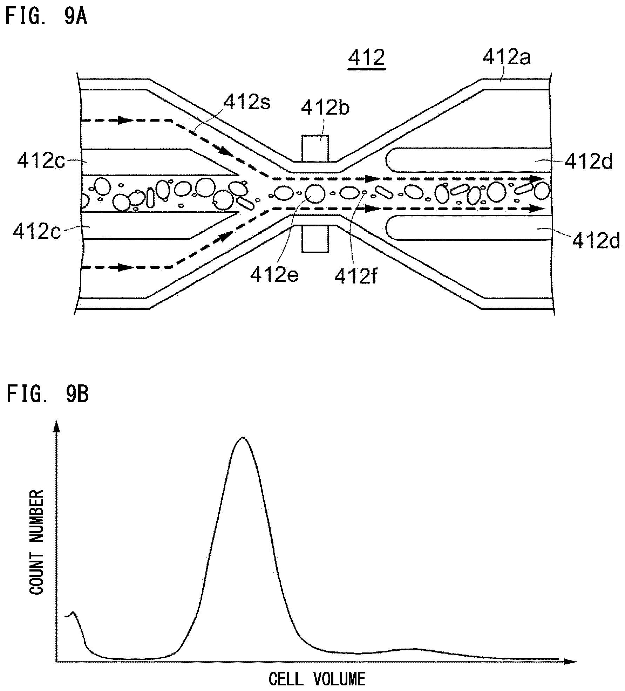

As shown in FIG. 9A, the red blood cell/platelet detector 412, which is a sheath flow-type electric resistance detector, includes: a chamber wall 412a; an aperture portion 412b for measuring an electric resistance of a cell; a sample nozzle 412c which supplies a sample; and a collection tube 412d which collets cells having passed through the aperture portion 412b. The space around the sample nozzle 412c and the collection tube 412d inside the chamber wall 412a is filled with the sheath liquid. Dashed line arrows indicated by the reference character 412s show the direction in which the sheath liquid flows. A red blood cell 412e and a platelet 412f discharged from the sample nozzle pass through the aperture portion 412b while being enveloped by the flow 412s of the sheath liquid. A constant DC voltage is applied to the aperture portion 412b, and control is performed such that a constant current flows while only the sheath liquid is flowing. A cell is less likely to allow electricity to pass therethrough, i.e., has a large electric resistance. Therefore, when a cell passes through the aperture portion 412b, the electric resistance is changed. Thus, at the aperture portion 412b, the number of times of passage of cells and the electric resistance at those times can be detected. The electric resistance increases in proportion to the volume of a cell. Therefore, the measurement unit information processing part 481 shown in FIG. 6 can calculate the volume of each cell having passed through the aperture portion 412b, render the count number of cells for each volume as a histogram shown in FIG. 9B, and display the histogram on the display/operation part 450 shown in FIG. 6, or send the histogram to the processing unit 300 via the bus 487 and the interface part 489. A signal regarding the electric resistance value is subjected to processing, similar to the processing performed on the signal obtained from the light described above, by the analogue processing part 420, the A/D converter 482, and the digital value calculation part 483 shown in FIG. 6, and is sent as a signal strength to the processing unit 300.

### <Second cell analyzer and measurement of biological sample in the second cell analyzer>

### (Configuration of measurement apparatus 2)

As a configuration example of the second cell analyzer 4000', an example of a block diagram when the measurement unit 500 is a flow cytometer for measuring a urine sample or a body fluid sample is shown.

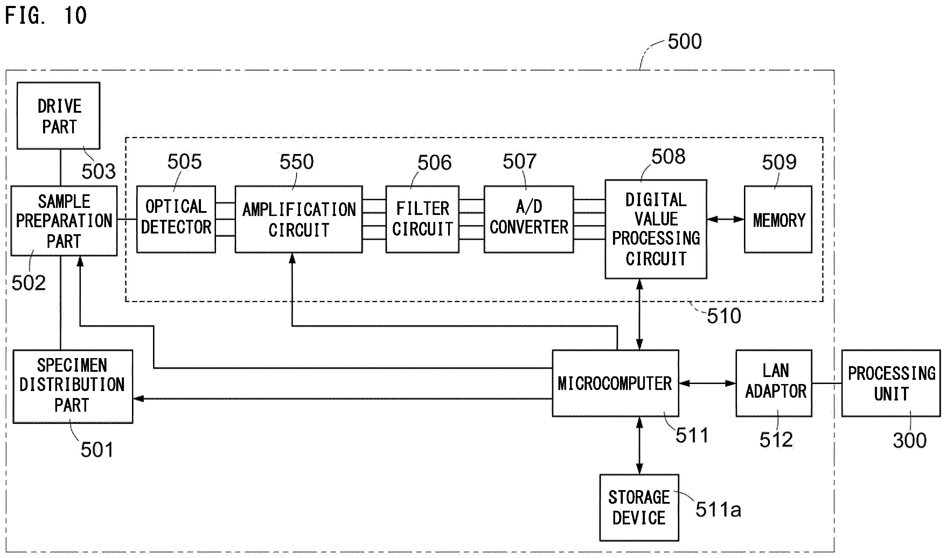

FIG. 10 is an example of a block diagram of the measurement unit 500. In FIG. 10, the measurement unit 500 includes: a specimen distribution part 501, a sample preparation part 502, and an optical detector 505; an amplification circuit 550 which amplifies an output signal (output signal amplified by a preamplifier) of the optical detector 505; a filter circuit 506 which performs filtering processing on an output signal from the amplification circuit 550; an A/D converter 507 which converts an output signal (analogue signal) of the filter circuit 506 to a digital value; a digital value processing circuit 508 which performs predetermined processing on the digital value; a memory 509 connected to the digital value processing circuit 508; a microcomputer 511 connected to the specimen distribution part 501, the sample preparation part 502, the amplification circuit 550, the digital value processing circuit 508, and a storage device 511a; and a LAN adaptor 12 connected to the microcomputer 511. The processing unit 300 is connected by a LAN cable to the measurement unit 500 via the LAN adaptor 12, and the processing unit 300 performs analysis of measurement data obtained in the measurement unit 500. The optical detector 505, the amplification circuit 550, the filter circuit 506, the A/D converter 507, the digital value processing circuit 508, and the memory 509 form an optical measurement part 510 which measures a measurement sample and generates measurement data.

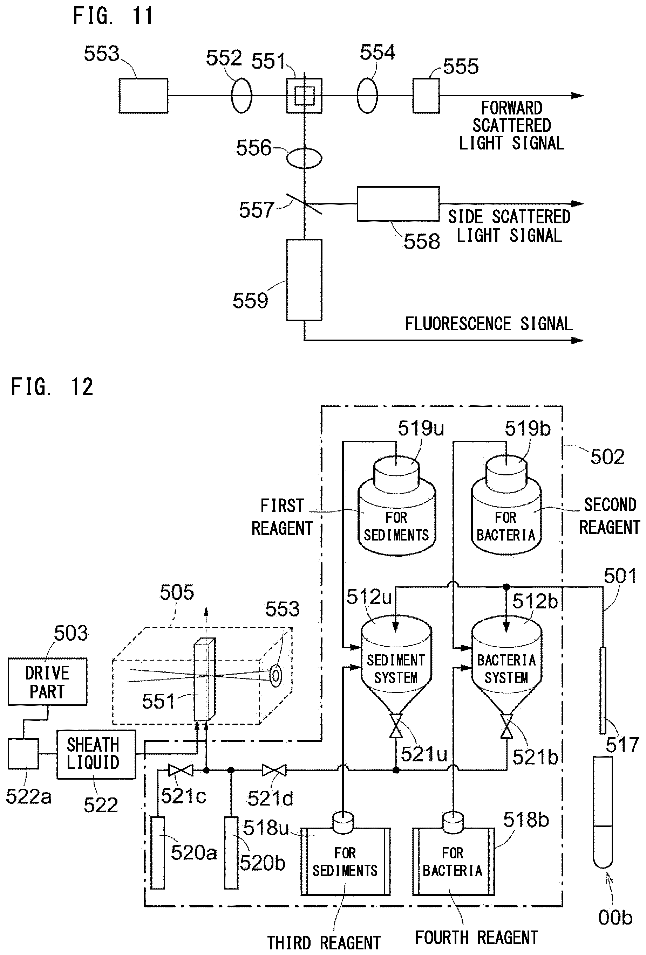

FIG. 11 shows a configuration of the optical detector 505 of the measurement unit 500. In FIG. 11, a condenser lens 552 condenses, to a flow cell 551, laser light emitted from a semiconductor laser light source 553 serving as a light source, and a condenser lens 554 condenses, to a forward scattered light receiving part 555, forward scattered light emitted from a solid component in a measurement sample. Another condenser lens 556 condenses, to a dichroic mirror 557, side scattered light and fluorescence emitted from the solid component. The dichroic mirror 557 reflects side scattered light to a side scattered light receiving part 558, and allows fluorescence to pass therethrough toward a fluorescence receiving part 559. These light signals reflect characteristics of the solid component in the measurement sample. The forward scattered light receiving part 555, the side scattered light receiving part 558, and the fluorescence receiving part 559 convert the light signals into electric signals, and output a forward scattered light signal, a side scattered light signal, and a fluorescence signal, respectively. These outputs are amplified by a preamplifier, and then subjected to the subsequent processing. With respect to each of the forward scattered light receiving part 555, the side scattered light receiving part 558, and the fluorescence receiving part 559, a low sensitivity output and a high sensitivity output can be switched, through switching of the drive voltage. The switching of sensitivity is performed by a microcomputer 11 described later. In the present embodiment, a photodiode may be used as the forward scattered light receiving part 555, photomultiplier tubes may be used as the side scattered light receiving part 558 and the fluorescence receiving part 559, or photodiodes may be used as the side scattered light receiving part 558 and the fluorescence receiving part 559. The fluorescence signal outputted from the fluorescence receiving part 559 is amplified by a preamplifier, and then provided to branched two signal channels. The two signal channels are each connected to the amplification circuit 550 described in FIG. 10. The fluorescence inputted to one of the signal channels is amplified by the amplification circuit 550 with high sensitivity.

### (Preparation of measurement sample)

FIG. 12 is a schematic diagram showing a function configuration of the sample preparation part 502 and the optical detector 505 shown in FIG. 10. The specimen distribution part 501 shown in FIG. 10 and FIG. 12 includes a suction tube 517 and a syringe pump. The specimen distribution part 501 suctions a specimen (urine or body fluid) 00b via the suction tube 517, and dispenses the specimen into the sample preparation part 502. The sample preparation part 502 includes a reaction chamber 512u and a reaction chamber 512b. The specimen distribution part 501 distributes a quantified measurement sample to each of the reaction chamber 512u and the reaction chamber 512b.

In the reaction chamber 512u, the distributed biological sample is mixed with a first reagent 519u as a diluent and a third reagent 518u that contains a dye. Due to the dye contained in the third reagent 518u, solid components in the specimen are stained. When the biological sample is urine, the sample prepared in the reaction chamber 512u is used as a first measurement sample for analyzing solid components in urine that are relatively large, such as red blood cells, white blood cells, epithelial cells, or tumor cells. When the biological sample is a body fluid, the sample prepared in the reaction chamber 512u is used as a third measurement sample for analyzing red blood cells in the body fluid.

Meanwhile, in the reaction chamber 512b, the distributed biological sample is mixed with a second reagent 519b as a diluent and a fourth reagent 518b that contains a dye. As described later, the second reagent 519b has a hemolytic action. Due to the dye contained in the fourth reagent 518b, solid components in the specimen are stained. When the biological sample is urine, the sample prepared in the reaction chamber 512b serves as a second measurement sample for analyzing bacteria in the urine. When the biological sample is a body fluid, the sample prepared in the reaction chamber 512b serves as a fourth measurement sample for analyzing nucleated cells (white blood cells and large cells) and bacteria in the body fluid.

A tube extends from the reaction chamber 512u to the flow cell 551 of the optical detector 505, whereby the measurement sample prepared in the reaction chamber 512u can be supplied to the flow cell 551. A solenoid valve 521u is provided at the outlet of the reaction chamber 512u. A tube extends also from the reaction chamber 512b, and this tube is connected to a portion of the tube extending from the reaction chamber 2u. Accordingly, the measurement sample prepared in the reaction chamber 512b can be supplied to the flow cell 551. A solenoid valve 521b is provided at the outlet of the reaction chamber 512u.

The tube extending from the reaction chamber 512u, 512b to the flow cell 551 is branched before the flow cell 551, and a branched tube is connected to a syringe pump 520a. A solenoid valve 521c is provided between the syringe pump 520a and the branched point.

Between the connection point of the tubes extending from the respective reaction chambers 512u, 512b and the branched point, the tube is further branched. A branched tube is connected to a syringe pump 520b. Between the branched point of the tube extending to the syringe pump 520b and the connection point, a solenoid valve 521d is provided.

The sample preparation part 502 has connected thereto a sheath liquid storing part 522 which stores a sheath liquid, and the sheath liquid storing part 522 is connected to the flow cell 551 by a tube. The sheath liquid storing part 522 has connected thereto a compressor 522a, and when the compressor 522a is driven, compressed air is supplied to the sheath liquid storing part 522, and the sheath liquid is supplied from the sheath liquid storing part 522 to the flow cell 551.

As for the two kinds of suspensions (measurement samples) prepared in the respective reaction chambers 512u, 512b, the suspension (the first measurement sample when the biological sample is urine, and the third measurement sample when the biological sample is a body fluid) of the reaction chamber 512u is first led to the optical detector 505, to form a thin flow enveloped by the sheath liquid in the flow cell 551, and laser light is applied to the thin flow. Then, in a similar manner, the suspension (the second measurement sample when the biological sample is urine, and the fourth measurement sample when the biological sample is a body fluid) of the reaction chamber 512b is led to the optical detector 505, to form a thin flow in the flow cell 551, and laser light is applied to the thin flow. Such operations are automatically performed by causing the solenoid valves 521a, 521b, 521c, 521d, a drive part 503, and the like to operate by control of the microcomputer 511 (controller) described later.

The first reagent to the fourth reagent are described in detail. The first reagent 519u is a reagent having a buffer as a main component, contains an osmotic pressure compensation agent so as to allow obtainment of a stable fluorescence signal without hemolyzing red blood cells, and is adjusted to have 100 to 600 mOsm/kg so as to realize an osmotic pressure suitable for classification measurement. Preferably, the first reagent 519u does not have a hemolytic action on red blood cells in urine.

Different from the first reagent 519u, the second reagent 519b has a hemolytic action. This is for facilitating passage of the later-described fourth reagent 518b through cell membranes of bacteria so as to promote staining. Further, this is also for contracting contaminants such as mucus fibers and red blood cell fragments. The second reagent 519b contains a surfactant in order to acquire a hemolytic action. As the surfactant, a variety of anionic, nonionic, and cationic surfactants can be used, but a cationic surfactant is particularly suitable. Since the surfactant can damage the cell membranes of bacteria, nucleic acids of bacteria can be efficiently stained by the dye contained in the fourth reagent 518b. As a result, bacteria measurement can be performed through a short-time staining process.

As still another embodiment, the second reagent 519b may acquire a hemolytic action not by a surfactant but by being adjusted to be acidic or to have a low pH. The second reagent 519b having a low pH means that the second reagent 519b has a lower pH than the first reagent 19u. When the first reagent 519u is neutral or weakly acidic to weakly alkaline, the second reagent 19b is acidic or strongly acidic. When the pH of the first reagent 519u is 6.0 to 8.0, the pH of the second reagent 519b is lower than that, and is preferably 2.0 to 6.0.

The second reagent 519b may contain a surfactant and be adjusted to have a low pH.

As still another embodiment, the second reagent 519b may acquire a hemolytic action by having a lower osmotic pressure than the first reagent 19u.

Meanwhile, the first reagent 519u does not contain any surfactant. In another embodiment, the first reagent 519u may contain a surfactant, but the kind and concentration thereof need to be adjusted so as not to hemolyze red blood cells. Therefore, preferably, the first reagent 519u does not contain the same surfactant as that of the second reagent 519b, or even if the first reagent 519u contains the same surfactant as that of the second reagent 519b, the concentration of the surfactant in the first reagent 519u is lower than that in the second reagent 519b.

The third reagent 518u is a staining reagent to be used in measurement of solid components in urine (red blood cells, white blood cells, epithelial cells, casts, or the like). As the dye contained in the third reagent 518u, a dye that stains membranes is selected, in order to also stain solid components that do not have nucleic acids. Preferably, the third reagent 518u contains an osmotic pressure compensation agent for the purpose of preventing hemolysis and for the purpose of obtaining a stable fluorescence intensity, and is adjusted to have 100 to 600 mOsm/kg so as to realize an osmotic pressure suitable for classification measurement. The cell membrane and nucleus (membrane) of solid components in urine are stained by the third reagent 18u. As the staining reagent containing a dye that stains membranes, a condensed benzene derivative is used, and a cyanine-based dye can be used, for example. The third reagent 18u stains not only cell membranes but also nuclear membranes. When the third reagent 518u is used in nucleated cells such as white blood cells and epithelial cells, the staining intensity in the cytoplasm (cell membrane) and the staining intensity in the nucleus (nuclear membrane) are combined, whereby the staining intensity becomes higher than in the solid components in urine that do not have nucleic acids. Accordingly, nucleated cells such as white blood cells and epithelial cells can be discriminated from solid components in urine that do not have nucleic acids such as red blood cells. As the third reagent, the reagents described in US Patent Publication No. 5891733 can be used. The third reagent 518u is mixed with urine or a body fluid, together with the first reagent 519u.

The fourth reagent 518b is a staining reagent that can accurately measure bacteria even when the specimen contains contaminants having sizes equivalent to those of bacteria and fungi. The fourth reagent 518b is described in detail in EP Patent Application Publication No. 1136563. As the dye contained in the fourth reagent 518b, a dye that stains nucleic acids is suitably used. As the staining reagent containing a dye that stains nuclei, the cyanine-based dyes of US Patent No. 7309581 can be used, for example. The fourth reagent 518b is mixed with urine or a specimen, together with the second reagent 519b.

Therefore, preferably, the third reagent 518u contains a dye that stains cell membranes, whereas the fourth reagent 518b contains a dye that stains nucleic acids. Solid components in urine may include those that do not have a nucleus, such as red blood cells. Therefore, by the third reagent 518u containing a dye that stains cell membranes, solid components in urine including those that do not have a nucleus can be detected. In addition, the second reagent can damage cell membranes of bacteria, and nucleic acids of bacteria and fungi can be efficiently stained by the dye contained in the fourth reagent 18b. As a result, bacteria measurement can be performed through a short-time staining process.

### [3. Waveform data analysis system 1]

### <Configuration of waveform data analysis system 1>

A third embodiment in the present embodiment relates to a waveform data analysis system.

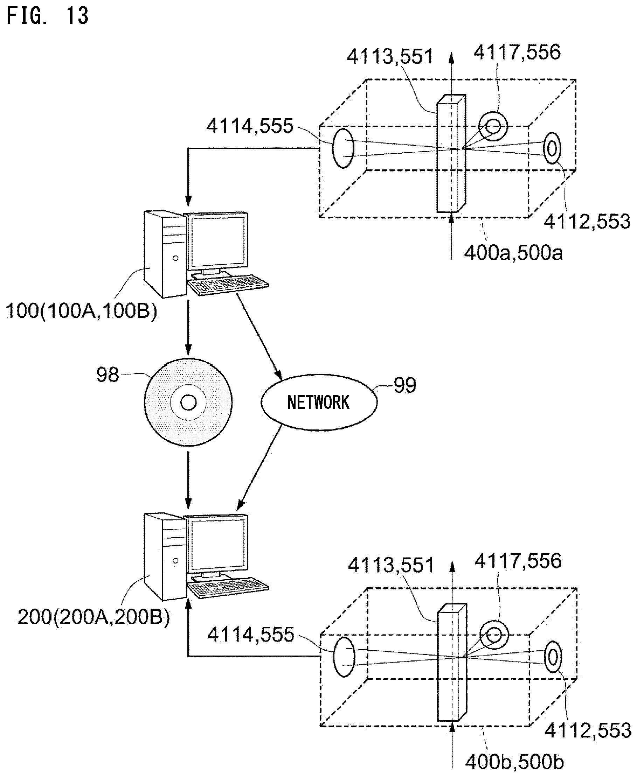

With reference to FIG. 13, a waveform data analysis system according to the third embodiment includes a deep learning apparatus 100A and an analyzer 200A. A vendor-side apparatus 100 operates as the deep learning apparatus 100A, and a user-side apparatus 200 operates as the analyzer 200A. The deep learning apparatus 100A causes the neural network 50 to learn by using training data, and provides a user with the deep learning algorithm 60 trained by the training data. The deep learning algorithm 60 configured as a learned neural network is provided from the deep learning apparatus 100A to the analyzer 200A through a storage medium 98 or a network 99. The analyzer 200A performs analysis of waveform data of an analysis target cell by using the deep learning algorithm 60 configured as a learned neural network.

The deep learning apparatus 100A is implemented as a general-purpose computer, for example, and performs a deep learning process on the basis of a flow chart described later. The analyzer 200A is implemented as a general-purpose computer, for example, and performs a waveform data analysis process on the basis of a flow chart described later. The storage medium 98 is a computer-readable non-transitory tangible storage medium such as a DVD-ROM or a USB memory, for example.

The deep learning apparatus 100A is connected to a measurement unit 400a or a measurement unit 500a. The configuration of the measurement unit 400a or the measurement unit 500a is the same as that of the measurement unit 400 or the measurement unit 500 described above. The deep learning apparatus 100A obtains training waveform data 70 obtained by the measurement unit 400a or the measurement unit 500a. The generation method of the training waveform data 70 is as described above. The analyzer 200A is also connected to the measurement unit 400b or the measurement unit 500b. The configuration of the measurement unit 400b or the measurement unit 500b is the same as that of the measurement unit 400 or the measurement unit 500 described above.

As shown in FIG. 7 and FIG. 11, the measurement unit 400 or the measurement unit 500 includes the flow cell 4113, 551. The measurement unit 400 or the measurement unit 500 sends a biological sample to the flow cell 4113, 551. A biological sample supplied to the flow cell 4113, 551 is irradiated with light from the light source 4112, 553, and forward scattered light, side scattered light, and side fluorescence emitted from a cell in the biological sample are detected by the light detectors 4116, 4121, 4122, 555, 558, 559. The light detectors 4116, 4121, 4122, 555, 558, 559 transmit signals to the vendor-side apparatus 100 or the user-side apparatus 200. The vendor-side apparatus 100 and the user-side apparatus 200 obtain waveform data of each of the forward scattered light, side scattered light, and side fluorescence detected by the light detectors 4116, 4121, 4122, 555, 558, 559.

### <Hardware configuration of deep learning apparatus>

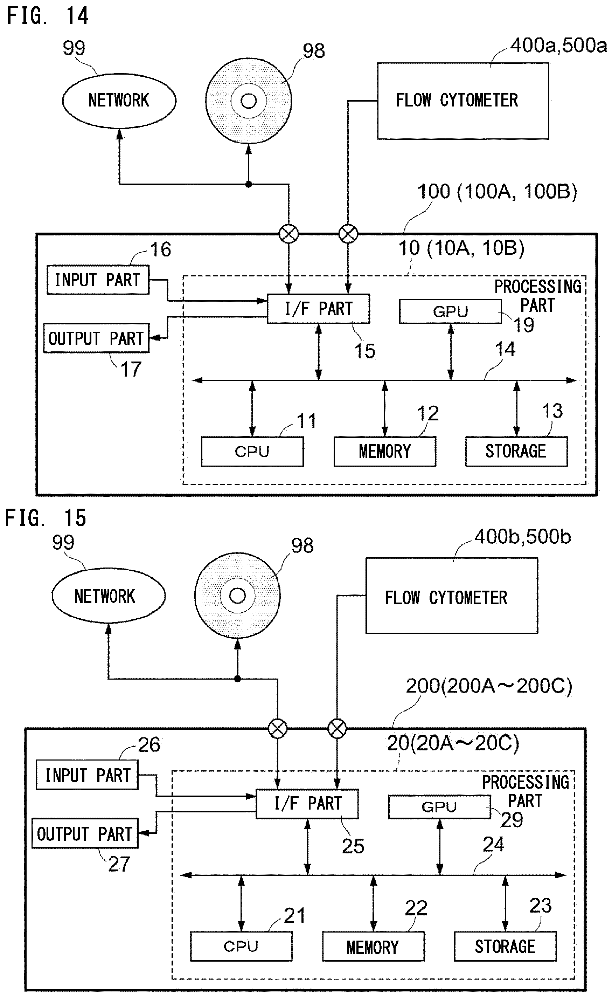

FIG. 14 shows an example of a block diagram of the vendor-side apparatus 100 (deep learning apparatus 100A, deep learning apparatus 100B). The vendor-side apparatus 100 includes a processing part 10 (10A, 10B), an input part 16, and an output part 17.

The processing part 10 includes: a CPU (Central Processing Unit) 11 which performs data processing described later; a memory 12 to be used as a work area for data processing; a storage 13 which stores a program and processing data described later; a bus 14 which transmits data between parts; an interface part 15 which inputs/outputs data with respect to an external apparatus; and a GPU (Graphics Processing Unit) 19. The input part 16 and the output part 17 are connected to the processing part 10 via the interface part 17. For example, the input part 16 is an input device such as a keyboard or a mouse, and the output part 17 is a display device such as a liquid crystal display. The GPU 19 functions as an accelerator that assists arithmetic processing (e.g., parallel arithmetic processing) performed by the CPU 11. That is, the processing performed by the CPU 11 described below also includes processing performed by the CPU 11 using the GPU 19 as an accelerator. Here, instead of the GPU 19, a chip that is suitable for calculation in a neural network may be installed. Examples of such a chip include FPGA (Field-Programmable Gate Array), ASIC (Application specific integrated circuit), and Myriad X (Intel).

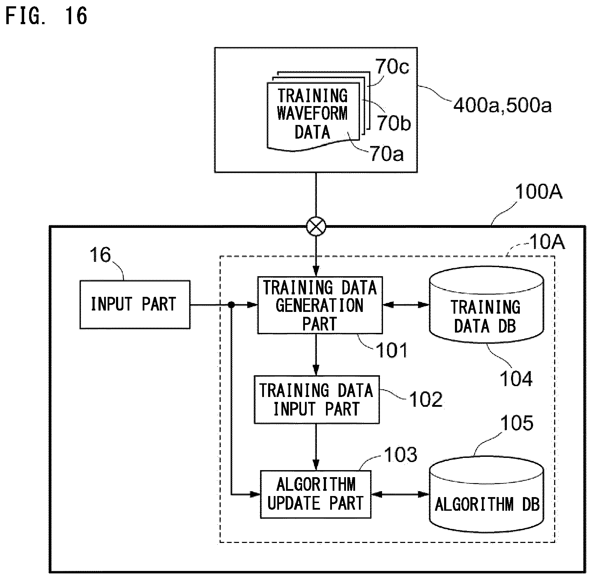

In order to perform the process of each step described below with reference to FIG. 16, the processing part 10 has previously stored, in the storage 13, a program and the neural network 50 before being trained according to the present invention, in an executable form, for example. The executable form is a form generated through conversion of a programming language by a compiler, for example. The processing part 10 uses the program stored in the storage 13, to perform training processes on the neural network 50 before being trained.

In the description below, unless otherwise specified, the processes performed by the processing part 10 mean processes performed by the CPU 11 on the basis of the program stored in the storage 13 or the memory 12, and the neural network 50. The CPU 11 temporarily stores necessary data (such as intermediate data being processed) using the memory 12 as a work area, and stores, as appropriate in the storage 13, data to be saved for a long time such as calculation results.

### <Hardware configuration of analyzer>

With reference to FIG. 15, the user-side apparatus 200 (analyzer 200A, analyzer 200B, analyzer 200C) includes a processing part 20 (20A, 20B, 20C), an input part 26, and an output part 27.

The processing part 20 includes: a CPU (Central Processing Unit) 21 which performs data processing described later; a memory 22 to be used as a work area for data processing; the storage 23 which stores a program and processing data described later; a bus 24 which transmits data between parts; an interface part 25 which inputs/outputs data with respect to an external apparatus; and a GPU (Graphics Processing Unit) 29. The input part 26 and the output part 27 are connected to the processing part 20 via the interface part 25. For example, the input part 26 is an input device such as a keyboard or a mouse, and the output part 27 is a display device such as a liquid crystal display. The GPU 29 functions as an accelerator that assists arithmetic processing (e.g., parallel arithmetic processing) performed by the CPU 21. That is, the processing performed by the CPU 21 described below also includes processing performed by the CPU 21 using the GPU 29 as an accelerator.

In order to perform the process of each step described in the waveform data analysis process below, the processing part 20 has previously stored, in the storage 23, a program and the deep learning algorithm 60 having a trained neural network structure according to the present invention, in an executable form, for example. The executable form is a form generated through conversion of a programming language by a compiler, for example. The processing part 20 uses the program and the deep learning algorithm 60 stored in the storage 23 to perform processes.

In the description below, unless otherwise specified, the processes performed by the processing part 20 mean, in actuality, processes performed by the CPU 21 of the processing part 20 on the basis of the program and the deep learning algorithm 60 stored in the storage 23 or the memory 22. The CPU 21 temporarily stores data (such as intermediate data being processed) using the memory 22 as a work area, and stores, as appropriate in the storage 23, data to be saved for a long time such as calculation results.

### <Function block and processing procedure>

### (Deep learning process)

With reference to FIG. 16, a processing part 10A of a deep learning apparatus 100A of the present embodiment includes a training data generation part 101, a training data input part 102, and an algorithm update part 103. These function blocks are realized when: a program for causing a computer to execute the deep learning process is installed in the storage 13 or the memory 12 of the processing part 10A shown in FIG. 14; and the program is executed by the CPU 11. A training data database (DB) 104 and an algorithm database (DB) 105 are stored in the storage 13 or the memory 12 of the processing part 10A.

The training waveform data 70a, 70b, 70c is obtained in advance by the measurement unit 400, 500, and is stored in advance in the storage 13 or the memory 12 of the processing part 10A. The deep learning algorithm 50 is stored in advance in the algorithm database 105 in association with the kind of cell to which each analysis target cell belongs, for example.

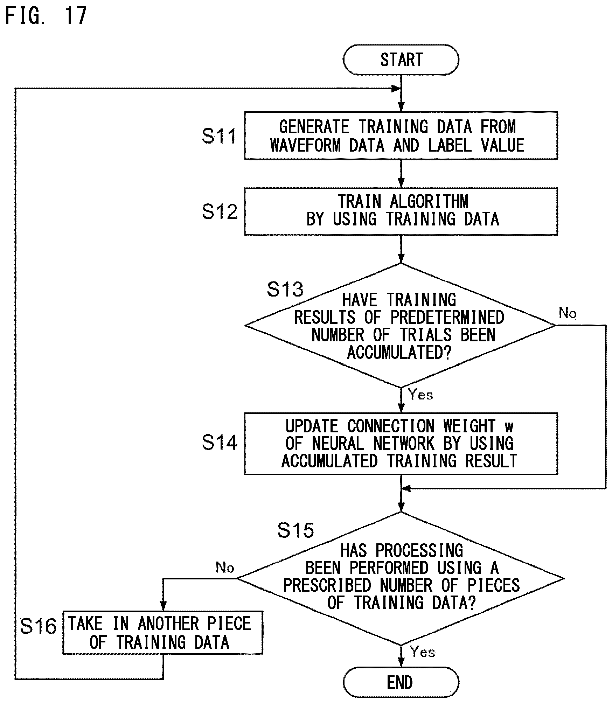

The processing part 10A of the deep learning apparatus 100A performs the process shown in FIG. 17. With reference to the function blocks shown in FIG. 16, the processes of steps S11, S14, and S16 shown in FIG. 17 are performed by the training data generation part 101. The process of step S12 is performed by the training data input part 102. The processes of steps S13 and S15 are performed by the algorithm update part 103.

With reference to FIG. 17, an example of the deep learning process performed by the processing part 10A is described.

First, the processing part 10A obtains the training waveform data 70a, 70b, 70c. The training waveform data 70a is waveform data of forward scattered light, the training waveform data 70b is waveform data of side scattered light, and the training waveform data 70c is waveform data of side fluorescence. The training waveform data 70a, 70b, 70c is obtained via the I/F part 15 in accordance with an operation by an operator, from the measurement unit 400, 500, from the storage medium 98, or via a network. When the training waveform data 70a, 70b, 70c is obtained, information regarding which kind of cell the training waveform data 70a, 70b, 70c indicates is also obtained. The information regarding which kind of cell is indicated may be associated with the training waveform data 70a, 70b, 70c, or may be inputted by the operator through the input part 16.

In step S11, the processing part 10A provides: information that indicates which kind of cell is indicated and that is associated with the training waveform data 70a, 70b, 70c; label values associated with the kinds of cells stored in the memory 12 or the storage 13; and a label value 77 that corresponds to the sequence data 76a, 76b, 76c obtained by synchronizing the sequence data 72a, 72b, 72c in terms of the time of obtainment of the waveform data of forward scattered light, side scattered light, and side fluorescence. Accordingly, the processing part 10A generates training data 75.

In step S12 shown in FIG. 17, the processing part 10A trains the neural network 50 by using the training data 75. The training result of the neural network 50 is accumulated every time training is performed using a plurality of pieces of training data 75.

In the cell type analysis method according to the present embodiment, a convolution neural network is used, and a stochastic gradient descent method is used. Therefore, in step S13, the processing part 10A determines whether or not training results of a previously-set predetermined number of trials have been accumulated. When the training results of the predetermined number of trials have been accumulated (YES), the processing part 10A advances to the process of step S14, and when the training results of the predetermined number of trials have not been accumulated (NO), the processing part 10A advances to the process of step S15.

Next, when the training results of the predetermined number of trials have been accumulated, the processing part 10A updates, in step S14, connection weights w of the neural network 50, by using the training results accumulated in step S12. In the cell type analysis method according to the present embodiment, since the stochastic gradient descent method is used, the connection weights w of the neural network 50 are updated at the stage where the learning results of the predetermined number of trials have been accumulated. Specifically, the process of updating the connection weights w is a process of performing calculation according to the gradient descent method, expressed by Formula 11 and Formula 12 described later.

In step S15, the processing part 10A determines whether or not the neural network 50 has been trained using a prescribed number of pieces of training data 75. When the training has been performed using the prescribed number of pieces of training data 75 (YES), the deep learning process ends.

When the neural network 50 has not been trained using the prescribed number of pieces of training data 75 (NO), the processing part 10A advances from step S15 to step S16, and performs the processes from step S11 to step S15 with respect to the next training waveform data 70.

In accordance with the processes described above, the neural network 50 is trained, whereby a deep learning algorithm 60 is obtained.

### (Structure of neural network)

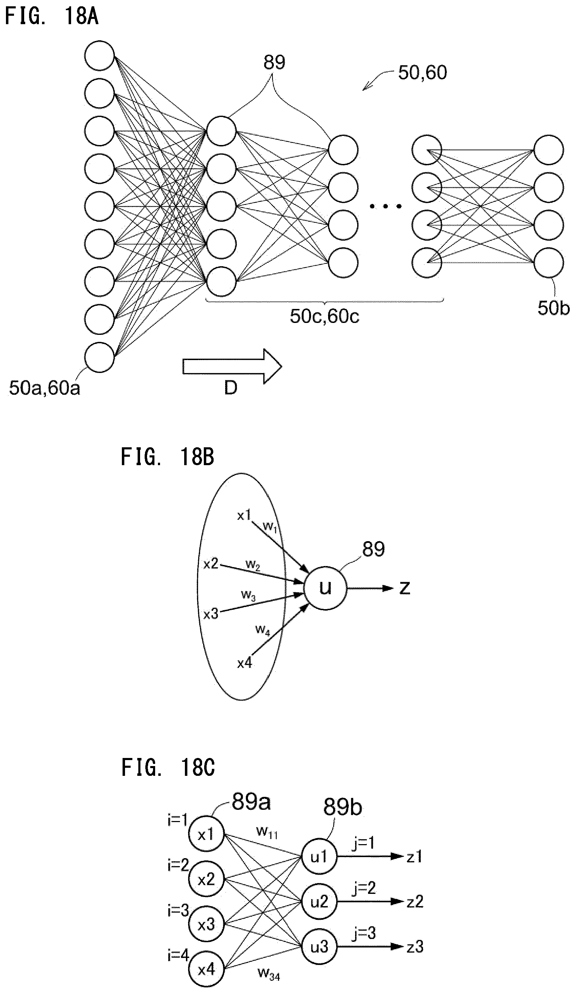

As described above, a convolution neural network is used in the present embodiment. FIG. 18A shows an example of the structure of the neural network 50. The neural network 50 includes the input layer 50a, the output layer 50b, and the middle layer 50c between the input layer 50a and the output layer 50b, and the middle layer 50c is composed of a plurality of layers. The number of layers forming the middle layer 50c can be, for example, 5 or greater, preferably 50 or greater, and more preferably 100 or greater.

In the neural network 50, a plurality of nodes 89 arranged in a layered manner are connected between the layers. Accordingly, information is propagated only in one direction indicated by an arrow D in FIG. 18A, from the input-side layer 50a to the output-side layer 50b.

### (Calculation at each node)

FIG. 18B is a schematic diagram showing calculation performed at each node. Each node 89 receives a plurality of inputs, and calculates one output (z). In the case of the example shown in FIG. 18B, the node 89 receives four inputs. The total input (u) received by the node 89 is expressed by Formula 1 below, for example. In the present embodiment, one-dimensional sequence data is used as each of the training data 75 and the analysis data 85. Therefore, when variables of the calculation formula correspond to two-dimensional matrix data, a process of converting the variables into one-dimensional ones is performed.

[Math 1] $u = {w}_{1} {x}_{1} + {w}_{2} {x}_{2} + {w}_{3} {x}_{3} + {w}_{4} {x}_{4} + b$ (Formula 1)

Each input is multiplied by a different weight. In Formula 1, b is a value called bias. The output (z) of the node serves as an output of a predetermined function f with respect to the total input (u) expressed by Formula 1, and is expressed by Formula 2 below. The function f is called an activation function.

[Math 2] $z = f \left(u\right)$ (Formula 2)

FIG. 18C is a schematic diagram illustrating calculation between nodes. In the neural network 50, with respect to the total input (u) expressed by Formula 1, nodes that output results (z) each expressed by Formula 2 are arranged in a layered manner. Outputs of the nodes of the previous layer serve as inputs to the nodes of the next layer. In the example shown in FIG. 18C, the outputs from nodes 89a in the left layer in FIG. 18C serve as inputs to nodes 89b in the right layer. Each node 89b in the right layer receives outputs from the respective nodes 89a in the left layer. The connection between each node 89a in the left layer and each node 89b in the right layer is multiplied by a different weight. When the respective outputs from the plurality of nodes 89a in the left layer are defined as x₁ to x₄, the inputs to the respective three nodes 89b in the right layer are expressed by Formula 3-1 to Formula 3-3 below. [Math 3] $\begin{matrix}{u}_{1} = {w}_{11} {x}_{1} + {w}_{12} {x}_{2} + {w}_{13} {x}_{3} + {w}_{14} {x}_{4} + {b}_{1} & \left(Formula 3-1\right) \\ {u}_{2} = {w}_{21} {x}_{1} + {w}_{22} {x}_{2} + {w}_{23} {x}_{3} + {w}_{24} {x}_{4} + {b}_{2} & \left(Formula 3-2\right) \\ {u}_{3} = {w}_{31} {x}_{1} + {w}_{32} {x}_{2} + {w}_{33} {x}_{3} + {w}_{34} {x}_{4} + {b}_{3} & \left(Formula 3-3\right)\end{matrix}$ When Formula 3-1 to Formula 3-3 are generalized, Formula 3-4 is obtained. Here, i=1, ... I, j = 1, ··· J.

[Math 4] ${u}_{j} = {\sum }_{i = 1}^{l} {w}_{ij} {x}_{i} + {b}_{j}$ (Formula 3-4) When Formula 3-4 is applied to the activation function, an output is obtained. The output is expressed by Formula 4 below.

[Math 5] ${z}_{j} = f \left({u}_{j}\right) \left(j=1,2,3\right)$ (Formula 4)

### (Activation function)

In the cell type analysis method according to the embodiment, a rectified linear unit function is used as the activation function. The rectified linear unit function is expressed by Formula 5 below.

[Math 6] $f \left(u\right) = max \left(u, 0\right)$ (Formula 5)

Formula 5 is a function obtained by setting u=0 to the part u<0 in the linear function with z=u. In the example shown in FIG. 18C, using Formula 5, the output from the node of j=1 is expressed by the formula below.

[Math 7] ${z}_{1} = max \left(\left({w}_{11}{x}_{1}+{w}_{12}{x}_{2}+{w}_{13}{x}_{3}+{w}_{14}{x}_{4}+{b}_{1}\right), 0\right)$

### (Neural network learning)

If the function expressed by use of a neural network is defined as y(x:w), the function y(x:w) varies when a parameter w of the neural network is varied. Adjusting the function y(x:w) such that the neural network selects a more suitable parameter w with respect to the input x is referred to as neural network learning. It is assumed that a plurality of pairs of an input and an output of the function expressed by use of the neural network have been provided. If a desirable output for an input x is defined as d, the pairs of the input/output are given as {(x₁,d₁), (x₂,d₂), ···, (xₙ,dₙ)}. The set of pairs each expressed as (x,d) is referred to as training data. Specifically, the set of pieces of waveform data (forward scattered light waveform data, side scattered light waveform data, fluorescence waveform data) shown in FIG. 2 is the training data shown in FIG. 2.

The neural network learning means adjusting the weight w such that, with respect to any input/output pair (xₙ,dₙ), the output y(xₙ:w) of the neural network when given an input xₙ, becomes as close to the output dₙ as much as possible. An error function is a measure for the closeness

[Math 8] $y \left({x}_{n}:w\right) \approx {d}_{n}$ between the training data and the function expressed by use of the neural network. The error function is also called a loss function. An error function E(w) used in the cell type analysis method according to the embodiment is expressed by Formula 6 below. Formula 6 is also called cross entropy.

[Math 9] $E \left(w\right) = - {\sum }_{n = 1}^{N} {\sum }_{k = 1}^{K} {d}_{nk} log {y}_{k} \left({x}_{n}; w\right)$ (Formula 6)

A method for calculating the cross entropy in Formula 6 is described. In the output layer 50b of the neural network 50 used in the cell type analysis method according to the embodiment, i.e., in the last layer of the neural network, an activation function for classifying inputs x into a finite number of classes according to the contents, is used. The activation function is called a softmax function, and expressed by Formula 7 below. It is assumed that, in the output layer 50b, the nodes are arranged by the same number as the number of classes k. It is assumed that the total input u of each node k (k=1, ···, K) of an output layer L is given as uₖ^{(L)} from the outputs of the previous layer L-1. Accordingly, the output of the k-th node in the output layer is expressed by Formula 7 below.

[Math 10] ${y}_{k} ≡ {z}_{k}^{\left(L\right)} = \frac{exp \left({u}_{k}^{\left(L\right)}\right)}{{\sum }_{j = 1}^{K} exp \left({u}_{j}^{\left(L\right)}\right)}$ (Formula 7)

Formula 7 is the softmax function. The sum of output y₁, ··· y_{K} determined by Formula 7 is always 1.

When each class is expressed as C₁, ···, C_{K}, output y_{K} of node k in the output layer L (i.e., uₖ^{(L)}) represents the probability that the given input x belongs to class C_{K}. Refer to Formula 8 below. The input x is classified into a class in which the probability expressed by Formula 8 becomes largest.

[Math 11] $p \left({C}_{k}\left|x\right.\right) = {y}_{k} = {z}_{k}^{\left(L\right)}$ (Formula 8)

In the neural network learning, a function expressed by the neural network is considered as a model of the posterior probability of each class, the likelihood of the weight w with respect to the training data is evaluated under such a probability model, and a weight w that maximizes the likelihood is selected.

It is assumed that target output dₙ by the softmax function of Formula 7 is 1 only if the output is a correct class, and otherwise, target output dₙ is 0. In a case where the target output is expressed in a vector format of dₙ=[dₙ₁, ···, d_{nK}], if, for example, the correct class of input xₙ is C₃, only target output dₙ₃ becomes 1, and the other target outputs become 0. When coding is performed in this manner, the posterior distribution is expressed by Formula 9 below.

[Math 12] $p \left(d\left|x\right.\right) = {\prod }_{k = 1}^{K} p {\left({C}_{k}\left|x\right.\right)}^{{d}_{k}}$ (Formula 9)

Likelihood L(w) of weight w with respect to the training data {(xₙ,dₙ)}(n=1, ···, N) is expressed by Formula 10 below. When the logarithm of likelihood L(w) is taken and the sign is inverted, the error function of Formula 6 is derived.

[Math 13] $\begin{matrix}L \left(w\right) = {\prod }_{n = 1}^{N} p \left({d}_{n}\left|{x}_{n}; w\right.\right) = {\prod }_{n = 1}^{N} {\prod }_{k = 1}^{K} p {\left({C}_{k}\left|{x}_{n}\right.\right)}^{{d}_{nk}} \\ = {\prod }_{n = 1}^{N} {\prod }_{k = 1}^{K} {\left({y}_{k}\left(x; w\right)\right)}^{{d}_{nk}}\end{matrix}$ (Formula 10)

Learning means minimizing error function E(w) calculated on the basis of the training data, with respect to parameter w of the neural network. In the cell type analysis method according to the embodiment, error function E(w) is expressed by Formula 6.

Minimizing error function E(w) with respect to parameter w has the same meaning as finding a local minimum point of function E(w). Parameter w is a weight of connection between nodes. The local minimum point of weight w is obtained by iterative calculation of repeatedly updating parameter w from an arbitrary initial value as a starting point. An example of such calculation is the gradient descent method.

In the gradient descent method, a vector expressed by Formula 11 below is used.
[Math 14] $∇ E = \frac{\partial E}{\partial w} = {\left[\frac{\partial E}{\partial {w}_{1}},…,\frac{\partial E}{\partial {w}_{M}}\right]}^{T}$ (Formula 11)

In the gradient descent method, a process of moving the value of current parameter w in the negative gradient direction (i.e., -∇E) is repeated many times. When the current weight is w^{(t)} and the weight after the moving is w^{(t+1)}, the calculation according to the gradient descent method is expressed by Formula 12 below. Value t means the number of times the parameter w is moved.

[Math 15] ${w}^{\left(t+1\right)} = {w}^{\left(t\right)} - ϵ ∇ E$ (Formula 12) [Math 16] $ϵ$

The above symbol is a constant that determines the magnitude of the update amount of parameter w, and is called a learning coefficient. As a result of repetition of the calculation expressed by Formula 12, error function E(w^{(t)}) decreases in association with increase of value t, and parameter w reaches a local minimum point.

It should be noted that the calculation according to Formula 12 may be performed on all of the training data (n=1, ···, N) or may be performed on only part of the training data. The gradient descent method performed on only part of the training data is called a stochastic gradient descent method. In the cell type analysis method according to the embodiment, the stochastic gradient descent method is used.

### (Waveform data analysis process)

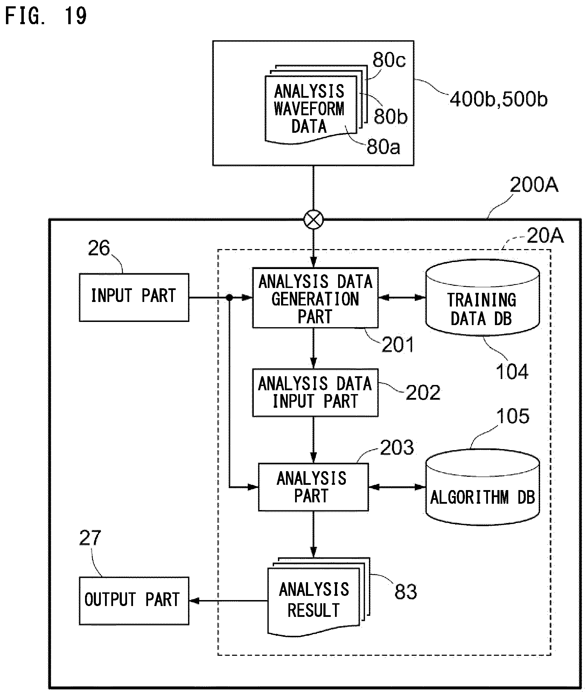

FIG. 19 shows a function block diagram of the analyzer 200A which performs the waveform data analysis process up to generation of an analysis result 83 from the analysis waveform data 80a, 80b, 80c. The processing part 20A of The analyzer 200A includes an analysis data generation part 201, an analysis data input part 202, and an analysis part 203. These function blocks are realized when: a program for causing a computer according to the present invention to execute the waveform data analysis process is installed in the storage 23 or the memory 22 of the processing part 20A shown in FIG. 15; and the program is executed by the CPU 21. The training data stored in a training data database (DB) 104 and the trained deep learning algorithm 60 stored in an algorithm database (DB) 105 are provided from the deep learning apparatus 100A through the storage medium 98 or the network 99, and are stored in the storage 23 or the memory 22 of the processing part 20A.

The analysis waveform data 80a, 80b, 80c is obtained by the measurement unit 400, 500 and is stored in the storage 23 or the memory 22 of the processing part 20A. The trained deep learning algorithm 60 including the trained connection weight w is associated with, for example, the kind of cell to which the analysis target cell belongs, and is stored in the algorithm database 105, and functions as a program module, which is part of the program that causes the computer to execute the waveform data analysis process. That is, the deep learning algorithm 60 is used by the computer including a CPU and a memory, and is used for calculating the probability of which kind of cell the analysis target cell corresponds to, and generating an analysis result 83 regarding the cell.

The generated analysis result 83 is outputted in the following manner. The CPU 21 of the processing part 20A causes the computer to function so as to execute calculation or processing of specific information according to the intended use. Specifically, the CPU 21 of the processing part 20A generates an analysis result 83 regarding the cell, by using the deep learning algorithm 60 stored in the storage 23 or the memory 22. The CPU 21 of the processing part 20A inputs the analysis data 85 into the input layer 60a, and outputs, from the output layer 61, the label value of the type of cell to which the analysis target cell belongs, i.e., the label value of the kind of the cell identified as the one to which the cell corresponding to the analysis waveform data belongs.

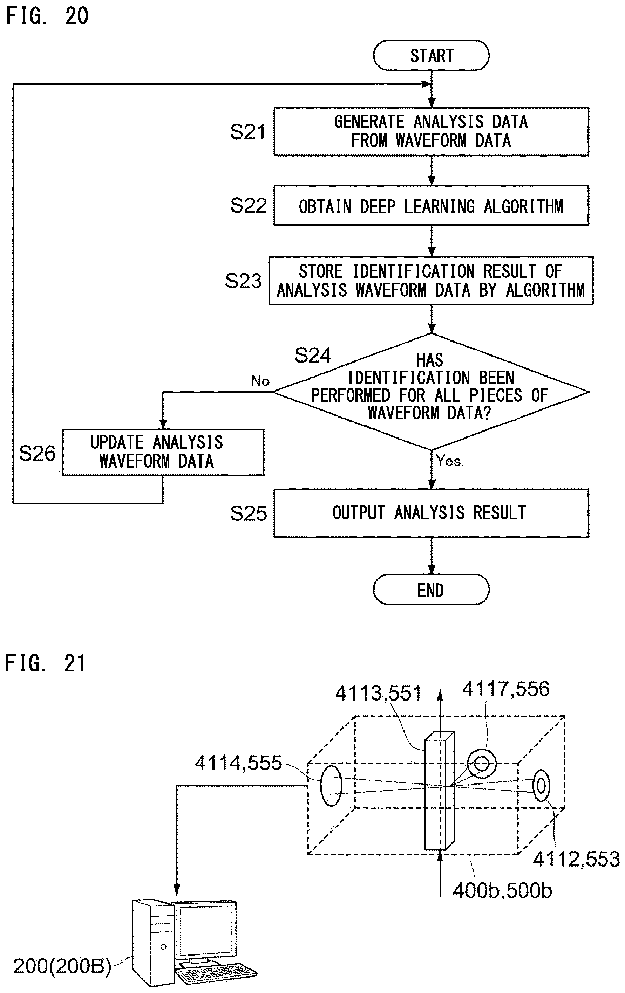

With reference to the flow chart shown in FIG. 20, the process of step S21 is performed by the analysis data generation part 201. The processes of steps S22, S23, S24, and S26 are performed by the analysis data input part 202. The process of step S25 is performed by the analysis part 203.

With reference to FIG. 20, an example of the waveform data analysis process, performed by the processing part 20A, up to generation of an analysis result 83 regarding the cell from the analysis waveform data 80a, 80b, 80c, is described.

First, the processing part 20A obtains analysis waveform data 80a, 80b, 80c. The analysis waveform data 80a, 80b, 80c is obtained via the I/F part 25, in accordance with an operation by the user or automatically, from the measurement unit 400, 500, from the storage medium 98, or via a network.

In step S21, from the sequences 82a, 82b, 82c, the processing part 20A generates an analysis result 83 regarding the cell in accordance with the procedure described in the analysis data generation method above.

Next, in step S22, the processing part 20A obtains the deep learning algorithm stored in the algorithm database 105. The order of steps S21 and S22 may be reversed.

Next, in step S23, the processing part 20A inputs the analysis result 83 regarding the cell, to the deep learning algorithm. In accordance with the procedure described in the waveform data analysis method above, the processing part 20A outputs a label value of the type of cell to which the analysis target cell from which the analysis waveform data 80a, 80b, 80c has been obtained has been determined to belong, on the basis of the deep learning algorithm. The processing part 20A stores this label value into the memory 22 or the storage 23.

In step S24, the processing part 20A determines whether the identification has been performed on all of the pieces of the analysis waveform data 80a, 80b, 80c obtained first. When the identification of all of the pieces of the analysis waveform data 80a, 80b, 80c has ended (YES), the processing part 20A advances to step S25, and outputs an analysis result including information 83 regarding each cell. When the identification of all of the pieces of the analysis waveform data 80a, 80b, 80c has not ended (NO), the processing part 20A advances to step S26, and performs the processes from step S22 to step S24, on the analysis waveform data 80a, 80b, 80c for which the identification has not yet been performed.

According to the present embodiment, it is possible to identify the kind of cell irrespective of the skill of the examiner.

### <Computer program>

The present embodiment includes a computer program, for waveform data analysis for analyzing the type of cell, that causes a computer to execute the processes of step S11 to S16 and/or S21 to S26.

Further, a certain embodiment of the present embodiment relates to a program product, such as a storage medium, having stored therein the computer program. That is, the computer program is stored in a storage medium such as a hard disk, a semiconductor memory device such as a flash memory, or an optical disk. The storage form of the program into the storage medium is not limited, as long as the vendor-side apparatus 100 and/or the user-side apparatus 200 can read the program. Preferably, the program is stored in the storage medium in a nonvolatile manner.

### [4. Waveform data analysis system 2]

### <Configuration of waveform data analysis system 2>

Another aspect of the waveform data analysis system is described.

FIG. 21 shows a configuration example of a second waveform data analysis system. The second waveform data analysis system includes a user-side apparatus 200, and the user-side apparatus 200 operates as an analyzer 200B of an integrated type. The analyzer 200B is implemented as a general-purpose computer, for example, and performs both the deep learning process and the waveform data analysis process described in the waveform data analysis system 1 above. That is, the second waveform data analysis system is a stand-alone-type system that performs deep learning and waveform data analysis on the user side. In the second waveform data analysis system, the integrated-type analyzer 200B provided on the user side has both functions of the deep learning apparatus 100A and the analyzer 200A according to the present embodiment.

In FIG. 21, the analyzer 200B is connected to the measurement unit 400b, 500b. The measurement unit 400 shown as an example in FIG. 5A and the measurement unit 500 shown as an example in FIG. 5B obtain the training waveform data 70a, 70b, 70c when the deep learning process is performed, and obtain the analysis waveform data 80a, 80b, 80c when the waveform data analysis process is performed.

### <Hardware configuration>

The hardware configuration of the analyzer 200B is the same as the hardware configuration of the user-side apparatus 200 shown in FIG. 15.

### <Function block and processing procedure>

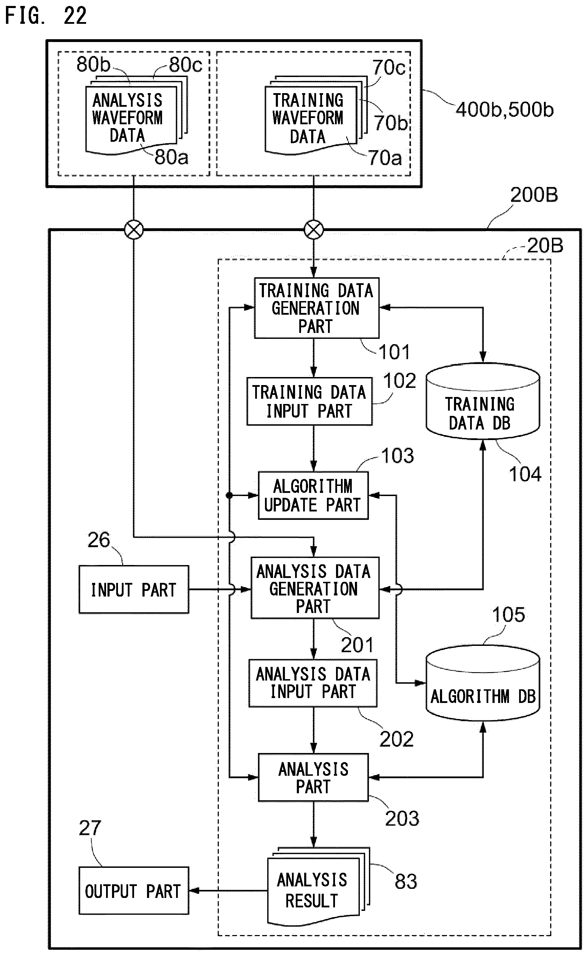

FIG. 22 shows a function block diagram of the analyzer 200B. The processing part 20B of the analyzer 200B includes a training data generation part 101, a training data input part 102, an algorithm update part 103, an analysis data generation part 201, an analysis data input part 202, an analysis part 203, and analysis results 83 regarding types of cells. These function blocks are realized when: a program for causing a computer to execute the deep learning process and the waveform data analysis process is installed in the storage 23 or the memory 22 of the processing part 20B, shown as an example in FIG. 15; and the program is executed by the CPU 21. A training data database (DB) 104 and an algorithm database (DB) 105 are stored in the storage 23 or the memory 22 of the processing part 20B, and both are used in common at the time of the deep learning and the waveform data analysis process. A deep learning algorithm 60 including the trained neural network is stored in advance in the algorithm database 105, in association with, for example, the kind of cell and the type of cell to which the analysis target cell belongs. The connection weight w is updated by the deep learning process, and the deep learning algorithm 60 is stored as a new deep learning algorithm 60 into the algorithm database 105. It is assumed that the training waveform data 70a, 70b, 70c has been obtained in advance by the measurement unit 400b, 500b as described above, and is stored in advance in the training data database (DB) 104 or in the storage 23 or the memory 22 of the processing part 20B. It is assumed that the analysis waveform data 80a, 80b, 80c of the specimen to be analyzed is obtained in advance by the measurement unit 400b, 500b, and is stored in advance in the storage 23 or the memory 22 of the processing part 20B.

The processing part 20B of the analyzer 200B performs the process shown in FIG. 17 at the time of the deep learning process, and performs the process shown in FIG. 20 at the time of the waveform data analysis process. With reference to the function blocks shown in FIG. 22, at the time of the deep learning process, the processes of steps S11, S15, and S16 are performed by the training data generation part 101. The process of step S12 is performed by the training data input part 102. The processes of steps S13 and S18 are performed by the algorithm update part 103. At the time of the waveform data analysis process, the process of step S21 is performed by the analysis data generation part 201. The processes of steps S22, S23, S24, and S26 are performed by the analysis data input part 202. The process of step S25 is performed by the analysis part 203.

The procedure of the deep learning process and the procedure of the waveform data analysis process that are performed by the analyzer 200B are similar to the procedures respectively performed by the deep learning apparatus 100A and the analyzer 200A. However, the analyzer 200B obtains the training waveform data 70a, 70b, 70c from the measurement unit 400b, 500b.

In the case of the analyzer 200B, the user can confirm the identification accuracy by the trained deep learning algorithm 60. Should the determination result by the deep learning algorithm 60 be different from the determination result according to the observation of the waveform data by the user, if the analysis waveform data 80a, 80b, 80c is used as the training data 70a, 70b, 70c, and the determination result according to the observation of the waveform data by the user is used as the label value 77, it is possible to train the deep learning algorithm again. Accordingly, the training efficiency of the deep learning algorithm 50 can be improved.

### [5. Waveform data analysis system 3]

### <Configuration of waveform data analysis system 3>

Another aspect of the waveform data analysis system is described.

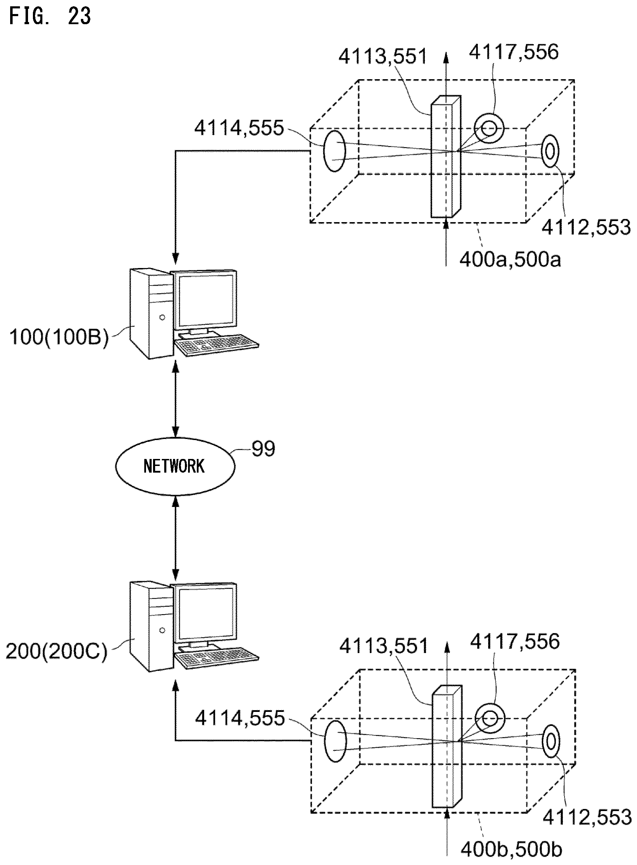

FIG. 23 shows a configuration example of a third waveform data analysis system. The third waveform data analysis system includes a vendor-side apparatus 100 and a user-side apparatus 200. The vendor-side apparatus 100 operates as an integrated-type analyzer 100B, and the user-side apparatus 200 operates as a terminal apparatus 200C. The analyzer 100B is implemented as a general-purpose computer, for example, and is a cloud-server-side apparatus that performs both the deep learning process and the waveform data analysis process described in the waveform data analysis system 1. The terminal apparatus 200C is implemented as a general-purpose computer, for example, and is a user-side terminal apparatus that transmits analysis waveform data 80a, 80b, 80c of the analysis target cell to the analyzer 100B through the network 99, and receives analysis results 83 from the analyzer 100B through the network 99.

In the third waveform data analysis system, the integrated-type analyzer 100B provided on the vendor side has both functions of the deep learning apparatus 100A and the analyzer 200A. Meanwhile, the third waveform data analysis system includes the terminal apparatus 200C, and provides the user-side terminal apparatus 200C with an input interface for the analysis waveform data 80a, 80b, 80c, and an output interface for the analysis result of waveform data. That is, the third waveform data analysis system is a cloud-service type system in which the vendor side that performs the deep learning process and the waveform data analysis process has an input interface for providing the analysis waveform data 80a, 80b, 80c to the user side, and an output interface for providing information 83 regarding cells to the user side. The input interface and the output interface may be integrated.

The analyzer 100B is connected to the measurement unit 400a, 500a, and obtains the training waveform data 70a, 70b, 70c obtained by the measurement unit 400a, 500a.

The terminal apparatus 200C is connected to the measurement unit 400b, 500b, and obtains the analysis waveform data 80a, 80b, 80c obtained by the measurement unit 400b, 500b.

### <Hardware configuration>

The hardware configuration of the analyzer 100B is the same as the hardware configuration of the vendor-side apparatus 100 shown in FIG. 14. The hardware configuration of the terminal apparatus 200C is the same as the hardware configuration of the user-side apparatus 200 shown in FIG. 15.

### <Function block and processing procedure>

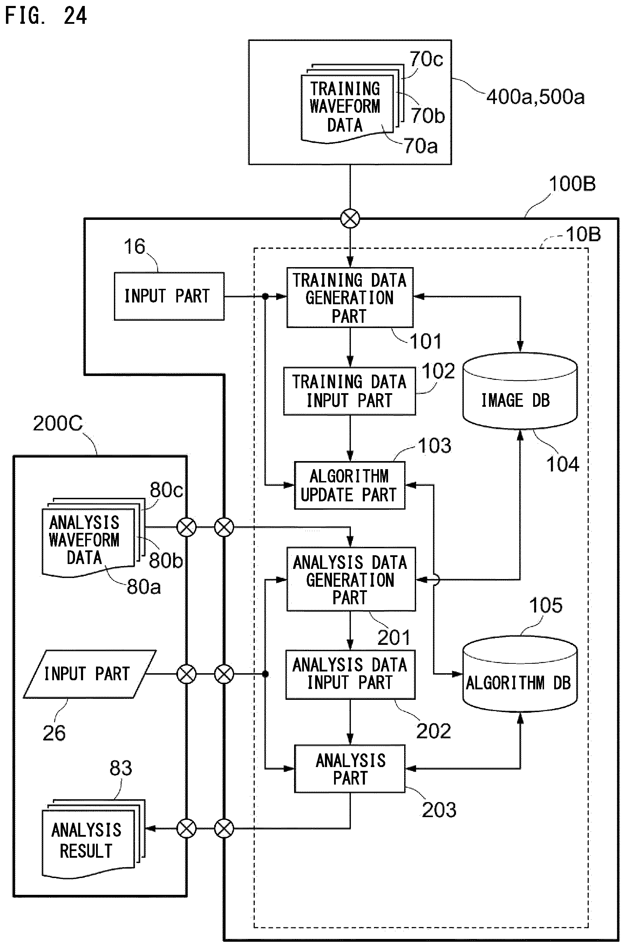

FIG. 24 shows a function block diagram of the analyzer 100B. A processing part 10B of the analyzer 100B includes a training data generation part 101, a training data input part 102, an algorithm update part 103, an analysis data generation part 201, an analysis data input part 202, and an analysis part 203. These function blocks are realized when: a program for causing a computer to execute the deep learning process and the waveform data analysis process is installed in the storage 13 or the memory 12 of the processing part 10B shown in FIG. 14; and the program is executed by the CPU 11. A training data database (DB) 104 and an algorithm database (DB) 105 are stored in the storage 13 or the memory 12 of the processing part 10B, and both are used in common at the time of the deep learning and the waveform data analysis process. A neural network 50 is stored in advance in the algorithm database 105, in association with, for example, the kind or type of cell to which the analysis target cell belongs, and the connection weight w is updated by the deep learning process, and is stored as the deep learning algorithm 60 into the algorithm database 105.

The training waveform data 70a, 70b, 70c is obtained in advance by the measurement unit 400a, 500a as described above, and is stored in advance in the training data database (DB) 104 or in the storage 13 or the memory 12 of the processing part 10B. It is assumed that the analysis waveform data 80a, 80b, 80c is obtained by the measurement unit 400b, 500b, and is stored in advance in the storage 23 or the memory 22 of the processing part 20C of the terminal apparatus 200C.

The processing part 10B of the analyzer 100B performs the process shown in FIG. 17 at the time of the deep learning process, and performs the process shown in FIG. 20 at the time of the waveform data analysis process. With reference to the function blocks shown in FIG. 24, at the time of the deep learning process, the processes of steps S11, S15, and S16 are performed by the training data generation part 101. The process of step S12 is performed by the training data input part 102. The processes of steps S13 and S18 are performed by the algorithm update part 103. At the time of the waveform data analysis process, the process of step S21 is performed by the analysis data generation part 201. The processes of steps S22, S23, S24, and S26 are performed by the analysis data input part 202. The process of step S25 is performed by the analysis part 203.

The procedure of the deep learning process and the procedure of the waveform data analysis process that are performed by the analyzer 100B are similar to the procedures respectively performed by the deep learning apparatus 100A and the analyzer 200A according to the present embodiment.

The processing part 10B receives the training waveform data 70a, 70b, 70c from the user-side terminal apparatus 200C, and generates training data 75 in accordance with steps S11 to S16 shown in FIG. 17.

In step S25 shown in FIG. 20, the processing part 10B transmits an analysis result including information 83 regarding cells, to the user-side terminal apparatus 200C. In the user-side terminal apparatus 200C, the processing part 20C outputs the received analysis result to the output part 27.

As described above, by transmitting the analysis waveform data 80a, 80b, 80c to the analyzer 100B, the user of the terminal apparatus 200C can obtain analysis results 83 regarding the types of cells, as an analysis result.

According to the analyzer 100B of the third embodiment, the user can use a discriminator without obtaining the training data database 104 and the algorithm database 105 from the deep learning apparatus 100A. Accordingly, a service of identifying the kinds of cells can be provided as a cloud service.

### [6. Other embodiments]

Although the outline and specific embodiments of the present invention have been described, the present invention is not limited to the outline and the embodiments described above.

In each place image data analysis system, the processing part 10A, 10B is realized as a single apparatus. However, the processing part 10A, 10B need not be a single apparatus. The CPU 11, the memory 12, the storage 13, the GPU 19, and the like may be provided at separate places and connected to each other through a network. The processing part 10A, 10B, the input part 16, the output part 17 also need not necessarily be provided at one place, and may be respectively provided at different places and communicably connected to each other through a network. This also applies to the processing part 20A, 20B, 20C.

In the first to third embodiments, the function blocks of the training data generation part 101, the training data input part 102, the algorithm update part 103, the analysis data generation part 201, the analysis data input part 202, and the analysis part 203 are executed by the single CPU 11 or the single CPU 21. However, these function blocks need not necessarily be executed by a single CPU, and may be executed in a distributed manner by a plurality of CPUs. These function blocks may be executed in a distributed manner by a plurality of GPUs, or may be executed in a distributed manner by a plurality of CPUs and a plurality of GPUs.

In the second and third embodiments, the program for performing the process of each step described in FIG. 17 and FIG. 20 is stored in advance in the storage 13, 23. Instead, the program may be installed into the processing part 10B, 20B from, for example, the computer-readable non-transitory tangible storage medium 98, such as a DVD-ROM or a USB memory. Alternatively, the processing part 10B, 20B may be connected to the network 99 and the program may be downloaded and installed via the network 99 from, for example, an external server (not shown).

In each waveform data analysis system, the input part 16, 26 is an input device such as a keyboard or a mouse, and the output part 17, 27 is realized as a display device such as a liquid crystal display. Instead of this, the input part 16, 26 and the output part 17, 27 may be integrated to be realized as a touch panel-type display device. Alternatively, the output part 17, 27 may be implemented as a printer or the like.

In each waveform data analysis system, the measurement unit 400a, 500a is directly connected to the deep learning apparatus 100A or the analyzer 100B. However, the flow cytometry 300 may be connected to the deep learning apparatus 100A or the analyzer 100B via the network 99. Similarly, although the flow cytometry 400 is directly connected to the analyzer 200A or the analyzer 200B, the measurement unit 400b, 500b may be connected to the analyzer 200A or the analyzer 200B via the network 99.

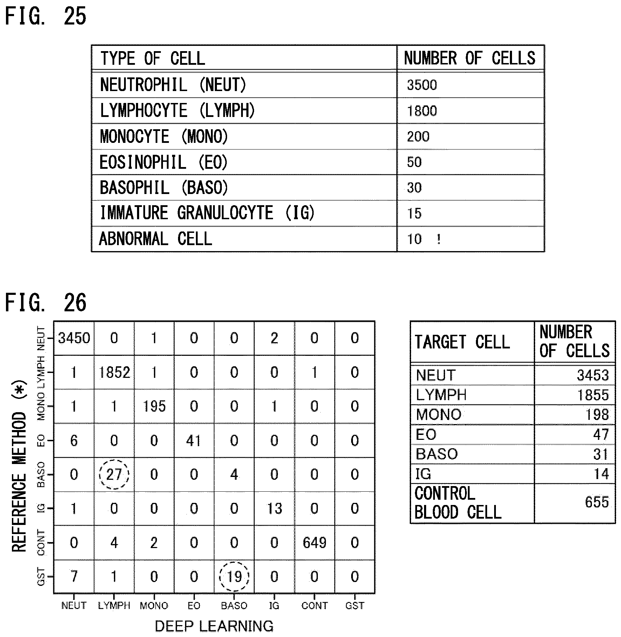

FIG. 25 shows an embodiment of the analysis result outputted to the output part 27. FIG. 25 shows the types, of cells contained in the biological sample measured by flow cytometry, that are provided with the label values shown in FIG. 3, and the number of cells of each type of cell. Instead of the display of the number of cells, or together with the display of the number of cells, the proportion (e.g., %) of each type of cell with respect to the total number of cells that have been counted, may be outputted. The count of the number of cells can be obtained by counting the number of label values (the number of the same label value) that correspond to each type of cell that has been outputted. In the output result, a warning indicating that abnormal cells are contained in the biological sample, may be outputted. FIG. 25 shows an example, but not limited thereto, in which an exclamation mark is provided as a warning in the column of the abnormal cell. Further, the distribution of each type of cell may be plotted as a scattergram, and the scattergram may be outputted. When the scattergram is outputted, for example, the highest values at the time of obtainment of signal strengths may be plotted, with the vertical axis representing the side fluorescence intensity and the horizontal axis representing the side scattered light intensity, for example.

### Example

### 1. Construction of deep learning model

Using Sysmex XN-1000, blood collected from a healthy individual was measured as a healthy blood sample, and XN CHECK Lv2 (control blood from Streck (having been subjected to processing such as fixation)) was measured as an unhealthy blood sample. As a fluorescence staining reagent, Fluorocell WDF manufactured by Sysmex Corporation was used. As a hemolytic agent, Lysercell WDF manufactured by Sysmex Corporation was used. For each cell contained in each specimen, waveform data of forward scattered light, side scattered light, and side fluorescence was obtained at 1024 points at a 10 nanosecond interval from the measurement start of forward scattered light. With respect to the healthy blood sample, waveform data of cells in blood collected from 8 healthy individuals was pooled as digital data. With respect to the waveform data of each cell, classification of neutrophil (NEUT), lymphocyte (LYMPH), monocyte (MONO), eosinophil (EO), basophil (BASO), and immature granulocyte (IG) was manually performed, and each piece of waveform data was provided with annotation (labelling) of the type of cell. The time point at which the signal strength of forward scattered light exceeded a threshold was defined as the measurement start time point, and the time points of obtainment of pieces of waveform data of forward scattered light, side scattered light, and side fluorescence were synchronized to each other, to generate training data. In addition, the control blood was provided with annotation "control blood-derived cell (CONT)". The training data was inputted to the deep learning algorithm to be learned by the deep learning algorithm.

With respect to blood cells of another healthy individual different from the healthy individual from whom the cell data having been learned was obtained, analysis waveform data was obtained by Sysmex XN-1000 in a manner similar to that for training data. Waveform data derived from the control blood was mixed, to create analysis data. With respect to this analysis data, blood cells derived from the healthy individual and blood cells derived from the control blood overlapped each other on the scattergram, and were not able to be discerned at all by a conventional method. This analysis data was inputted to a constructed deep learning algorithm, and data of the types of individual cells was obtained.

FIG. 26 shows the result as a mix matrix. The horizontal axis represents the determination result by the constructed deep learning algorithm, and the vertical axis represents the determination result manually (reference method) obtained by a human. With respect to the determination result by the constructed deep learning algorithm, although slight confusions were observed between basophil and lymphocyte and between basophil and ghost, the determination result by the constructed deep learning algorithm exhibited a matching rate of 98.8% with the determination result by the reference method.

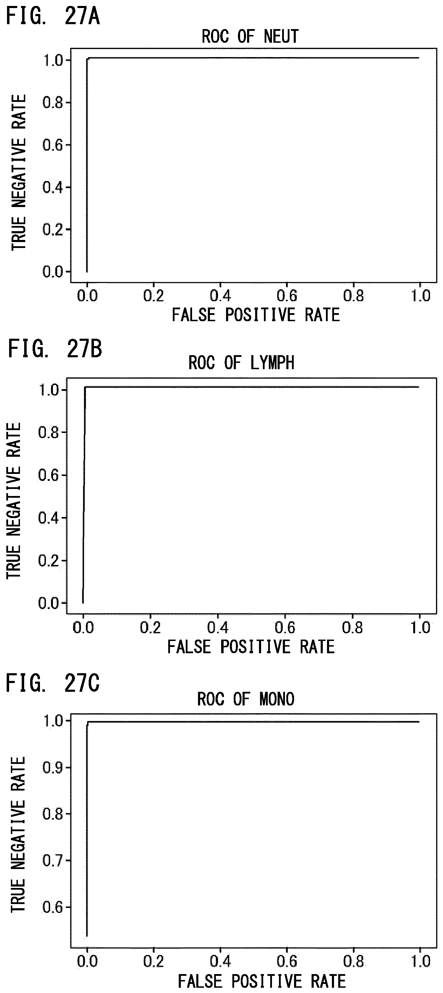

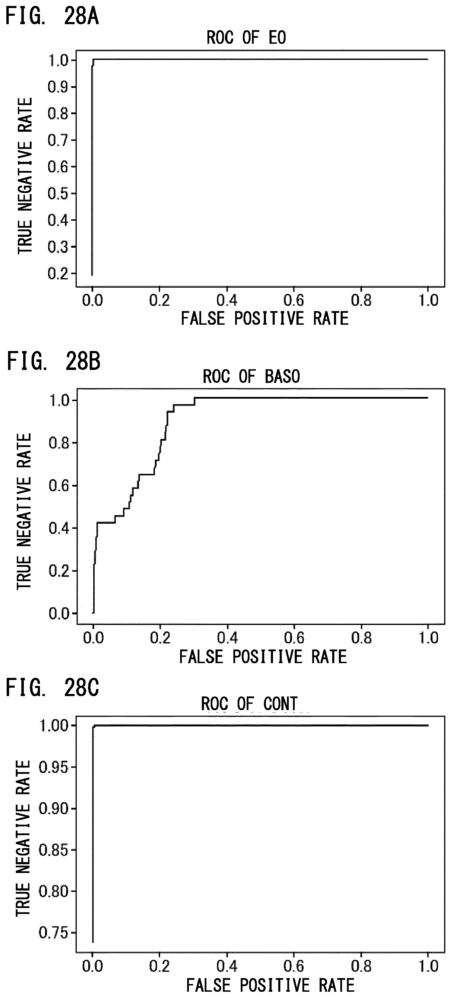

Next, with respect to each type of cell, ROC analysis was performed, and sensitivity and specificity were evaluated. FIG. 27A shows an ROC curve of neutrophil, FIG. 27B shows an ROC curve of lymphocyte, FIG. 27C shows an ROC curve of monocyte, FIG. 28A shows an ROC curve of neutrophil, FIG. 28B shows an ROC curve of basophil, and FIG. 28C shows an ROC curve of control blood (CONT). Sensitivity and specificity were, respectively, 99.5% and 99.6% for neutrophil, 99.4% and 99.5% for lymphocyte, 98.5% and 99.9% for monocyte, 97.9% and 99.8% for eosinophil, 71.0% and 81.4% for basophil, and 99.8% and 99.6% for control blood (CONT). These were good results.

From the result above, it has been clarified that type of cell can be determined by using the deep learning algorithm on the basis of signals obtained from a cell contained in a biological sample and on the basis of waveform data.

Further, there are cases where, when unhealthy blood cells such as a control blood are mixed with healthy blood cells, it is difficult to make determination by a conventional scattergram method. However, it has been shown that, when the deep learning algorithm of the present embodiment is used, even when unhealthy blood cells are mixed with healthy blood cells, it is possible to make determination about these cells.

### DESCRIPTION OF THE REFERENCE CHARACTERS

- 4000, 4000': cell analyzer
- 10: processing part
- 50: deep learning algorithm before being trained
- 50a: input layer
- 50b: output layer
- 60: trained deep learning algorithm
- 75: training data
- 80: analysis data
- 83: analysis result regarding type of cell

## Claims

1. A cell analysis method for analyzing cells contained in a biological sample, by using a deep learning algorithm (60) having a neural network structure, the cell analysis method comprising:
causing the cells to flow through a flow cell;
obtaining signal strengths of light signals which are signals obtained by light being applied to each of the individual cells passing through the flow cell, the light signals being a side scattered light signal, a forward scattered light signal, and a fluorescence signal, and inputting, into the deep learning algorithm (60), numerical data corresponding to the obtained signal strengths regarding each of the individual cells; and
on the basis of a result outputted from the deep learning algorithm (60), determining, for each cell, a type of the cell for which the signal strengths have been obtained, wherein
the numerical data corresponding to the signal strength inputted to the deep learning algorithm (60) includes information obtained by combining strengths of signals of the side scattered light signal, the forward scattered light signal, and the fluorescence signal that have been obtained for each cell.

2. The cell analysis method of claim 1, wherein
from the individual cells passing through a predetermined position in the flow cell, the signal strength is obtained, for each of the cells, at a plurality of time points in a time period while the cell is passing through the predetermined position, and
each obtained signal strength is stored in association with information regarding a corresponding time point at which the signal strength has been obtained.

3. The cell analysis method of claim 2, wherein
the obtaining of the signal strength at the plurality of time points is started at a time point at which the signal strength of each of the individual cells has reached a predetermined value, and ends after a predetermined time period after the start of the obtaining of the signal strength.

4. The cell analysis method of claim 2, wherein
the predetermined position is a position where the light is applied to each cell in the flow cell.

5. The analysis method of claim 1, wherein
the light is laser light.

6. The cell analysis method of claim 1, wherein
the deep learning algorithm (60) calculates, for each cell, a probability that the cell for which the signal strength has been obtained belongs to each of a plurality of types of cells associated with an output layer (60b) of the deep learning algorithm (60).

7. The cell analysis method of claim 6, wherein
the deep learning algorithm (60) outputs a label value of a type of a cell that has a highest probability that the cell for which the signal strength has been obtained belongs thereto.

8. The cell analysis method of claim 7, wherein
on the basis of the label value of the type of the cell that has the highest probability that the cell for which the signal strength has been obtained belongs thereto, the number of cells that belong to each of the plurality of types of cells is counted, and a result of the counting is outputted, or
on the basis of the label value of the type of the cell that has the highest probability that the cell for which the signal strength has been obtained belongs thereto, a proportion of cells that belong to each of the plurality of types of cells is calculated, and a result of the calculation is outputted.

9. The cell analysis method of any one of claims 1 to 8, wherein
the biological sample is a blood sample.

10. The cell analysis method of claim 9, wherein
the type of a cell includes at least one type selected from a group consisting of neutrophil, lymphocyte, monocyte, eosinophil, and basophil.

11. The cell analysis method of claim 10, wherein
the type of a cell includes at least one type selected from the group consisting of (a) and (b) below:
(a) immature granulocyte; and
(b) at least one type of abnormal cell selected from the group consisting of tumor cell, lymphoblast, plasma cell, atypical lymphocyte, reactive lymphocyte, nucleated erythrocyte selected from proerythroblast, basophilic erythroblast, polychromatic erythroblast, orthochromatic erythroblast, promegaloblast, basophilic megaloblast, polychromatic megaloblast, and orthochromatic megaloblast, and megakaryocyte.

12. The cell analysis method of claim 11, wherein
the type of a cell includes abnormal cell, and
when there is a cell that has been determined to be an abnormal cell by the deep learning algorithm (60), information indicating that an abnormal cell is contained in the biological sample is outputted.

13. The cell analysis method of any one of claims 1 to 8, wherein the biological sample is urine.

14. A method for training a deep learning algorithm (50) having a neural network structure for analyzing cells contained in a biological sample, the method comprising:
causing the cells to flow through a flow cell, and inputting, as first training data (75) to an input layer (50a) of the deep learning algorithm (50), numerical data corresponding to obtained signal strengths of light signals which are signals obtained by light being applied to each of the individual cells passing through the flow cell, the light signals being a side scattered light signal, a forward scattered light signal, and a fluorescence signal; and
inputting, as second training data (75) to the deep learning algorithm (50), information of a type of a cell that corresponds to the cell for which the signal strengths have been obtained, wherein
the numerical data corresponding to the signal strength inputted to the deep learning algorithm (50) includes information obtained by combining strengths of signals of the side scattered light signal, the forward scattered light signal, and the fluorescence signal that have been obtained for each cell.

15. A cell analyzer (4000) configured to determine a type of each of cells contained in a biological sample, by using a deep learning algorithm (60) having a neural network structure,
the cell analyzer (4000) comprising a measurement part (400) and a processing part (10), wherein
the measurement part (400) is configured to:
obtain, when the cells pass through a flow cell, signal strengths of light signals which are signals obtained by light being applied to each of the individual cells passing through the flow cell, the light signals being a side scattered light signal, a forward scattered light signal, and a fluorescence signal; and
the processing part (10) is configured to:
input, to the deep learning algorithm (60), numerical data corresponding to the obtained signal strengths regarding each of the individual cells; and
on the basis of a result outputted from the deep learning algorithm (60), determine, for each cell, a type of the cell for which the signal strengths have been obtained, wherein
the numerical data corresponding to the signal strength inputted to the deep learning algorithm (60) includes information obtained by combining strengths of signals of the side scattered light signal, the forward scattered light signal, and the fluorescence signal that have been obtained for each cell.

16. A training apparatus for training a deep learning algorithm (50) having a neural network structure for analyzing cells contained in a biological sample,
the training apparatus comprising a measurement part (400) and a processing part (10), wherein
the measurement part (400) is configured to:
cause the cells to flow through a flow cell, and input, as first training data (75) to an input layer (50a) of the deep learning algorithm (50), numerical data corresponding to signal strengths of light signals which are signals obtained by light being applied to each of the individual cells passing through the flow cell, the light signals being a side scattered light signal, a forward scattered light signal, and a fluorescence signal; and
the processing part (10) is configured to:
input, as second training data (75) to the deep learning algorithm (50), information of a type of a cell that corresponds to the cell for which the signal strengths have been obtained, wherein
the numerical data corresponding to the signal strength inputted to the deep learning algorithm (60) includes information obtained by combining strengths of signals of the side scattered light signal, the forward scattered light signal, and the fluorescence signal that have been obtained for each cell.

17. A computer program for analyzing cells contained in a biological sample, by using a deep learning algorithm (60) having a neural network structure,
the computer program being configured to cause a measurement part (400) to execute a process comprising:
a step of causing the cells to flow through a flow cell, and obtaining signal strengths of light signals which are signals obtained by light being applied to each of the individual cells passing through the flow cell, the light signals being a side scattered light signal, a forward scattered light signal, and a fluorescence signal;
the computer program being further configured to cause a processing part (10) to execute a process comprising:
a step of inputting, to the deep learning algorithm (60), numerical data corresponding to the obtained signal strengths regarding each of the individual cells; and
a step of, on the basis of a result outputted from the deep learning algorithm (60), determining, for each cell, a type of the cell for which the signal strengths have been obtained, wherein
the numerical data corresponding to the signal strength inputted to the deep learning algorithm (60) includes information obtained by combining strengths of signals of the side scattered light signal, the forward scattered light signal, and the fluorescence signal that have been obtained for each cell.

18. A computer program for training a deep learning algorithm (50) having a neural network structure for analyzing cells contained in a biological sample,
the computer program being configured to cause a measurement part (400) to execute a process comprising:
a step of causing the cells to flow through a flow cell, and inputting, as first training data (75) to an input layer (50a) of the deep learning algorithm (50), numerical data corresponding to obtained signal strengths of light signals which are signals obtained by light being applied to each of the individual cells passing through the flow cell, the light signals being a side scattered light signal, a forward scattered light signal, and a fluorescence signal; and
the computer program being configured to cause a processing part (10) to execute a process comprising: a step of inputting, as second training data (75) to the deep learning algorithm (50), information of a type of a cell that corresponds to the cell for which the signal strengths have been obtained, wherein
the numerical data corresponding to the signal strength inputted to the deep learning algorithm (50) includes information obtained by combining strengths of signals of the side scattered light signal, the forward scattered light signal, and the fluorescence signal that have been obtained for each cell.

## Patentansprüche

1. Ein Zellanalyseverfahren zum Analysieren von in einer biologischen Probe enthaltenen Zellen unter Verwendung eines Deep-Learning-Algorithmus (60) mit einer neuronalen Netzwerkstruktur, wobei das Zellanalyseverfahren umfasst:
Bewirken, dass die Zellen durch eine Durchflusszelle fließen;
Erhalten von Signalstärken von Lichtsignalen, bei denen es sich um Signale handelt, die dadurch erhalten werden, dass Licht auf jede der einzelnen Zellen angewendet wird, die durch die Durchflusszelle fließen, wobei die Lichtsignale ein Seitwärtsstreulichtsignal, ein Vorwärtsstreulichtsignal und ein Fluoreszenzsignal sind, und Eingeben numerischer Daten in den Deep-Learning-Algorithmus (60), die den erhaltenen Signalstärken in Bezug auf jede der einzelnen Zellen entsprechen; und
auf der Grundlage eines aus dem Deep-Learning-Algorithmus (60) ausgegebenen Ergebnisses Bestimmen eines Typs der Zelle für jede Zelle, für die die Signalstärken erhalten wurden,
wobei
die numerischen Daten, die der in den Deep-Learning-Algorithmus (60) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

2. Das Zellanalyseverfahren nach Anspruch 1, wobei
von den einzelnen Zellen, die eine vorbestimmte Position in der Durchflusszelle passieren, die Signalstärke für jede der Zellen zu mehreren Zeitpunkten in einer Zeitspanne erhalten wird, während die Zelle die vorbestimmte Position passiert, und
jede erhaltene Signalstärke in Verbindung mit Informationen bezüglich eines entsprechenden Zeitpunkts gespeichert wird, zu dem die Signalstärke erhalten wurde.

3. Das Zellanalyseverfahren nach Anspruch 2, wobei
das Erhalten der Signalstärke zu den mehreren Zeitpunkten zu einem Zeitpunkt gestartet wird, zu dem die Signalstärke jeder der einzelnen Zellen einen vorbestimmten Wert erreicht hat, und nach einer vorbestimmten Zeitspanne nach dem Start des Erhaltens der Signalstärke endet.

4. Das Zellanalyseverfahren nach Anspruch 2, wobei
die vorbestimmte Position eine Position ist, an der das Licht auf jede Zelle in der Durchflusszelle angewendet wird.

5. Das Analyseverfahren nach Anspruch 1, wobei
das Licht Laserlicht ist.

6. Das Zellanalyseverfahren nach Anspruch 1, wobei
der Deep-Learning-Algorithmus (60) für jede Zelle eine Wahrscheinlichkeit berechnet, dass die Zelle, für die die Signalstärke erhalten wurde, zu jedem einer Vielzahl von Zelltypen gehört, die einer Ausgabeschicht (60b) des Deep-Learning-Algorithmus (60) zugeordnet sind.

7. Das Zellanalyseverfahren nach Anspruch 6, wobei
der Deep-Learning-Algorithmus (60) einen Etikettenwert eines Typs einer Zelle ausgibt, bei der die höchste Wahrscheinlichkeit besteht, dass die Zelle, für die die Signalstärke erhalten wurde, dazu gehört.

8. Das Zellanalyseverfahren nach Anspruch 7, wobei
auf der Grundlage des Etikettenwerts des Typs der Zelle, bei der die höchste Wahrscheinlichkeit besteht, dass die Zelle, für die die Signalstärke erhalten wurde, dazu gehört, die Anzahl von Zellen, die zu jedem der Vielzahl von Zelltypen gehören, gezählt wird und ein Ergebnis der Zählung ausgegeben wird, oder
auf der Grundlage des Etikettenwerts des Typs der Zelle, bei der die höchste Wahrscheinlichkeit besteht, dass die Zelle, für die die Signalstärke erhalten wurde, dazu gehört, ein Anteil von Zellen berechnet wird, die zu jedem der Vielzahl von Zelltypen gehören, und ein Ergebnis der Berechnung ausgegeben wird.

9. Das Zellanalyseverfahren nach einem der Ansprüche 1 bis 8, wobei
die biologische Probe eine Blutprobe ist.

10. Das Zellanalyseverfahren nach Anspruch 9, wobei
der Zelltyp mindestens einen Typ umfasst, der aus einer Gruppe ausgewählt ist, die aus Neutrophilen, Lymphozyten, Monozyten, Eosinophilen und Basophilen besteht.

11. Das Zellanalyseverfahren nach Anspruch 10, wobei
der Zelltyp mindestens einen Typ umfasst, der aus der Gruppe ausgewählt ist, die aus den folgenden (a) und (b) besteht:
(a) unreifen Granulozyten; und
(b) mindestens einem Typ abnormer Zelle, ausgewählt aus der Gruppe bestehend aus Tumorzelle, Lymphoblast, Plasmazelle, atypischem Lymphozyten, reaktivem Lymphozyten, kernhaltigem Erythrozyten ausgewählt aus Proerythroblast, basophilem Erythroblast, polychromatischem Erythroblast, orthochromatischem Erythroblast, Promegaloblast, basophilem Megaloblast, polychromatischem Megaloblast und orthochromatischem Megaloblast sowie Megakaryozyten.

12. Das Zellanalyseverfahren nach Anspruch 11, wobei
der Zelltyp eine abnorme Zelle umfasst, und
wenn es eine Zelle gibt, die durch den Deep-Learning-Algorithmus (60) als abnorme Zelle bestimmt wurde, Informationen ausgegeben werden, die anzeigen, dass eine abnorme Zelle in der biologischen Probe enthalten ist.

13. Das Zellanalyseverfahren nach einem der Ansprüche 1 bis 8, wobei die biologische Probe Urin ist.

14. Ein Verfahren zum Trainieren eines Deep-Learning-Algorithmus (50) mit einer neuronalen Netzwerkstruktur zum Analysieren von in einer biologischen Probe enthaltenen Zellen, wobei das Verfahren umfasst:
Bewirken, dass die Zellen durch eine Durchflusszelle fließen, und Eingeben von numerischen Daten, die den erhaltenen Signalstärken von Lichtsignalen entsprechen, bei denen es sich um Signale handelt, die dadurch erhalten werden, dass Licht auf jede der einzelnen Zellen angewendet wird, die durch die Durchflusszelle fließen, als erste Trainingsdaten (75) in eine Eingabeschicht (50a) des Deep-Learning-Algorithmus (50), wobei die Lichtsignale ein Seitwärtsstreulichtsignal, ein Vorwärtsstreulichtsignal und ein Fluoreszenzsignal sind; und
Eingeben von Informationen über einen Zelltyp, der der Zelle entspricht, für die die Signalstärken erhalten wurden, als zweite Trainingsdaten (75) in den Deep-Learning-Algorithmus (50), wobei
die numerischen Daten, die der in den Deep-Learning-Algorithmus (50) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

15. Ein Zellanalysator (4000), der so konfiguriert ist, dass er unter Verwendung eines Deep-Learning-Algorithmus (60) mit einer neuronalen Netzwerkstruktur einen Typ von jeder in einer biologischen Probe enthaltenen Zellen bestimmt,
wobei der Zellanalysator (4000) einen Messteil (400) und einen Verarbeitungsteil (10) umfasst, wobei
der Messteil (400) für Folgendes konfiguriert ist:
Erhalten von Signalstärken von Lichtsignalen, wenn die Zellen durch eine Durchflusszelle passieren, bei denen es sich um Signale handelt, die dadurch erhalten werden, dass Licht auf jede der einzelnen Zellen angewendet wird, die durch die Durchflusszelle passieren, wobei die Lichtsignale ein Seitwärtsstreulichtsignal, ein Vorwärtsstreulichtsignal und ein Fluoreszenzsignal sind; und
der Verarbeitungsteil (10) für Folgendes konfiguriert ist:
Eingeben numerischer Daten in den Deep-Learning-Algorithmus (60), die den erhaltenen Signalstärken in Bezug auf jede der einzelnen Zellen entsprechen; und
auf der Grundlage eines aus dem Deep-Learning-Algorithmus (60) ausgegebenen Ergebnisses Bestimmen eines Typs der Zelle für jede Zelle, für die die Signalstärken erhalten wurden,
wobei
die numerischen Daten, die der in den Deep-Learning-Algorithmus (60) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

16. Eine Trainingsvorrichtung zum Trainieren eines Deep-Learning-Algorithmus (50) mit einer neuronalen Netzwerkstruktur zum Analysieren von in einer biologischen Probe enthaltenen Zellen,
wobei die Trainingsvorrichtung einen Messteil (400) und einen Verarbeitungsteil (10) umfasst, wobei
der Messteil (400) für Folgendes konfiguriert ist:
Bewirken, dass die Zellen durch eine Durchflusszelle fließen, und Eingeben von numerischen Daten, die Signalstärken von Lichtsignalen entsprechen, bei denen es sich um Signale handelt, die dadurch erhalten werden, dass Licht auf jede der einzelnen Zellen angewendet wird, die durch die Durchflusszelle fließen, als erste Trainingsdaten (75) in eine Eingabeschicht (50a) des Deep-Learning-Algorithmus (50), wobei die Lichtsignale ein Seitwärtsstreulichtsignal, ein Vorwärtsstreulichtsignal und ein Fluoreszenzsignal sind; und
der Verarbeitungsteil (10) für Folgendes konfiguriert ist:
Eingeben von Informationen über einen Zelltyp, der der Zelle entspricht, für die die Signalstärken erhalten wurden, als zweite Trainingsdaten (75) in den Deep-Learning-Algorithmus (50), wobei
die numerischen Daten, die der in den Deep-Learning-Algorithmus (60) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

17. Ein Computerprogramm zum Analysieren von in einer biologischen Probe enthaltenen Zellen unter Verwendung eines Deep-Learning-Algorithmus (60) mit einer neuronalen Netzwerkstruktur,
wobei das Computerprogramm so konfiguriert ist, dass es einen Messteil (400) veranlasst, einen Prozess auszuführen, der Folgendes umfasst:
einen Schritt, bei dem bewirkt wird, dass die Zellen durch eine Durchflusszelle fließen, und Signalstärken von Lichtsignalen erhalten werden, bei denen es sich um Signale handelt, die dadurch erhalten werden, dass Licht auf jede der einzelnen Zellen angewendet wird, die durch die Durchflusszelle fließen, wobei die Lichtsignale ein Seitwärtsstreulichtsignal, ein Vorwärtsstreulichtsignal und ein Fluoreszenzsignal sind;
wobei das Computerprogramm ferner so konfiguriert ist, dass es einen Verarbeitungsteil (10) veranlasst, einen Prozess auszuführen, der Folgendes umfasst:
einen Schritt des Eingebens numerischer Daten, die den erhaltenen Signalstärken in Bezug auf jede der einzelnen Zellen entsprechen, in den Deep-Learning-Algorithmus (60); und
einen Schritt des Bestimmens, auf der Grundlage eines aus dem Deep-Learning-Algorithmus (60) ausgegebenen Ergebnisses, eines Typs der Zelle für jede Zelle, für die die Signalstärken erhalten wurden, wobei
die numerischen Daten, die der in den Deep-Learning-Algorithmus (60) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

18. Ein Computerprogramm zum Trainieren eines Deep-Learning-Algorithmus (50) mit einer neuronalen Netzwerkstruktur zum Analysieren von in einer biologischen Probe enthaltenen Zellen,
wobei das Computerprogramm so konfiguriert ist, dass es einen Messteil (400) veranlasst, einen Prozess auszuführen, der Folgendes umfasst:
einen Schritt des Bewirkens, dass die Zellen durch eine Durchflusszelle fließen, und des Eingebens numerischer Daten, die erhaltenen Signalstärken von Lichtsignalen entsprechen, bei denen es sich um Signale handelt, die dadurch erhalten werden, dass Licht auf jede der einzelnen Zellen angewendet wird, die durch die Durchflusszelle fließen, als erste Trainingsdaten (75) in eine Eingabeschicht (50a) des Deep-Learning-Algorithmus (50), wobei die Lichtsignale ein Seitwärtsstreulichtsignal, ein Vorwärtsstreulichtsignal und ein Fluoreszenzsignal sind; und
wobei das Computerprogramm so konfiguriert ist, dass es einen Verarbeitungsteil (10) veranlasst, einen Prozess auszuführen, der Folgendes umfasst: einen Schritt des Eingebens von Informationen über einen Zelltyp, der der Zelle entspricht, für die die Signalstärken erhalten wurden, als zweite Trainingsdaten (75) in den Deep-Learning-Algorithmus (50), wobei
die numerischen Daten, die der in den Deep-Learning-Algorithmus (50) eingegebenen Signalstärke entsprechen, Informationen umfassen, die durch Kombinieren der Stärken von Signalen des Seitwärtsstreulichtsignals, des Vorwärtsstreulichtsignals und des Fluoreszenzsignals erhalten werden, die für jede Zelle erhalten wurden.

## Revendications

1. Un procédé d'analyse de cellules destiné à analyser des cellules contenues dans un échantillon biologique, à l'aide d'un algorithme d'apprentissage profond (60) présentant une structure de réseau neuronal, le procédé d'analyse de cellules comprenant :
le fait de faire s'écouler les cellules à travers une cuve à circulation ;
l'obtention d'intensités de signaux de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence, et l'introduction, dans l'algorithme d'apprentissage profond (60), de données numériques correspondant aux intensités de signaux obtenues concernant chacune des cellules individuelles ; et
sur la base d'un résultat délivré par l'algorithme d'apprentissage profond (60), la détermination, pour chaque cellule, d'un type de la cellule pour laquelle les intensités de signaux ont été obtenues,
dans lequel
les données numériques correspondant à l'intensité de signal introduite dans l'algorithme d'apprentissage profond (60) comprennent des informations obtenues en combinant les intensités de signaux du signal de lumière diffusée latéralement, du signal de lumière diffusée vers l'avant et du signal de fluorescence qui ont été obtenues pour chaque cellule.

2. Le procédé d'analyse de cellules de la revendication 1, dans lequel
à partir des cellules individuelles passant à travers une position prédéterminée dans la cuve à circulation, l'intensité de signal est obtenue, pour chacune des cellules, à une pluralité d'instants dans une période temporelle pendant que la cellule passe à travers la position prédéterminée, et
chaque intensité de signal obtenue est stockée en association avec des informations concernant un instant correspondant auquel l'intensité de signal a été obtenue.

3. Le procédé d'analyse de cellules de la revendication 2, dans lequel
l'obtention de l'intensité de signal à la pluralité d'instants est démarrée à un instant auquel l'intensité de signal de chacune des cellules individuelles a atteint une valeur prédéterminée, et se termine après une période temporelle prédéterminée après le démarrage de l'obtention de l'intensité de signal.

4. Le procédé d'analyse de cellules de la revendication 2, dans lequel
la position prédéterminée est une position où la lumière est appliquée à chaque cellule dans la cuve à circulation.

5. Le procédé d'analyse de la revendication 1, dans lequel
la lumière est une lumière laser.

6. Le procédé d'analyse de cellules de la revendication 1, dans lequel
l'algorithme d'apprentissage profond (60) calcule, pour chaque cellule, une probabilité que la cellule pour laquelle l'intensité de signal a été obtenue appartienne à chacun d'une pluralité de types de cellules associés à une couche de sortie (60b) de l'algorithme d'apprentissage profond (60).

7. Le procédé d'analyse de cellules de la revendication 6, dans lequel
l'algorithme d'apprentissage profond (60) délivre une valeur d'étiquette d'un type d'une cellule qui présente une probabilité la plus élevée que la cellule pour laquelle l'intensité de signal a été obtenue y appartienne.

8. Le procédé d'analyse de cellules de la revendication 7, dans lequel
sur la base de la valeur d'étiquette du type de la cellule qui présente la probabilité la plus élevée que la cellule pour laquelle l'intensité de signal a été obtenue y appartienne, le nombre de cellules qui appartiennent à chacun de la pluralité de types de cellules est compté, et un résultat du comptage est délivré, ou
sur la base de la valeur d'étiquette du type de la cellule qui présente la probabilité la plus élevée que la cellule pour laquelle l'intensité de signal a été obtenue y appartienne, une proportion de cellules qui appartiennent à chacun de la pluralité de types de cellules est calculée, et un résultat du calcul est délivré.

9. Le procédé d'analyse de cellules de l'une quelconque des revendications 1 à 8, dans lequel
l'échantillon biologique est un échantillon de sang.

10. Le procédé d'analyse de cellules de la revendication 9, dans lequel
le type d'une cellule comprend au moins un type sélectionné dans un groupe constitué d'un neutrophile, d'un lymphocyte, d'un monocyte, d'un éosinophile et d'un basophile.

11. Le procédé d'analyse de cellules de la revendication 10, dans lequel
le type d'une cellule comprend au moins un type sélectionné dans le groupe constitué de (a) et (b) ci-dessous :
(a) un granulocyte immature ; et
(b) au moins un type de cellule anormale sélectionné dans le groupe constitué d'une cellule tumorale, d'un lymphoblaste, d'un plasmocyte, d'un lymphocyte atypique, d'un lymphocyte réactif, d'un érythrocyte nucléé sélectionné parmi un proérythroblaste, un érythroblaste basophile, un érythroblaste polychromatique, un érythroblaste orthochromatique, un promégaloblaste, un mégaloblaste basophile, un mégaloblaste polychromatique et un mégaloblaste orthochromatique, et d'un mégacaryocyte.

12. Le procédé d'analyse de cellules de la revendication 11, dans lequel
le type d'une cellule comprend une cellule anormale, et
lorsqu'il existe une cellule qui a été déterminée comme étant une cellule anormale par l'algorithme d'apprentissage profond (60), des informations indiquant qu'une cellule anormale est contenue dans l'échantillon biologique sont délivrées.

13. Le procédé d'analyse de cellules de l'une quelconque des revendications 1 à 8, dans lequel l'échantillon biologique est de l'urine.

14. Un procédé d'entraînement d'un algorithme d'apprentissage profond (50) présentant une structure de réseau neuronal pour l'analyse de cellules contenues dans un échantillon biologique, le procédé comprenant :
le fait de faire s'écouler les cellules à travers une cuve à circulation, et l'introduction, en tant que premières données d'entraînement (75) vers une couche d'entrée (50a) de l'algorithme d'apprentissage profond (50), de données numériques correspondant à des intensités de signaux obtenues de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence ; et
l'introduction, en tant que secondes données d'entraînement (75) vers l'algorithme d'apprentissage profond (50), d'informations d'un type d'une cellule qui correspond à la cellule pour laquelle les intensités de signaux ont été obtenues, dans lequel
les données numériques correspondant à l'intensité de signal introduite dans l'algorithme d'apprentissage profond (50) comprennent des informations obtenues en combinant les intensités de signaux du signal de lumière diffusée latéralement, du signal de lumière diffusée vers l'avant et du signal de fluorescence qui ont été obtenues pour chaque cellule.

15. Un analyseur de cellules (4000) configuré pour déterminer un type de chacune des cellules contenues dans un échantillon biologique, à l'aide d'un algorithme d'apprentissage profond (60) présentant une structure de réseau neuronal,
l'analyseur de cellules (4000) comprenant une partie de mesure (400) et une partie de traitement (10), dans lequel
la partie de mesure (400) est configurée pour :
obtenir, lorsque les cellules passent à travers une cuve à circulation, des intensités de signaux de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence ; et
la partie de traitement (10) est configurée pour :
introduire, dans l'algorithme d'apprentissage profond (60), des données numériques correspondant aux intensités de signaux obtenues concernant chacune des cellules individuelles ; et
sur la base d'un résultat délivré par l'algorithme d'apprentissage profond (60), déterminer, pour chaque cellule, un type de la cellule pour laquelle les intensités de signaux ont été obtenues,
dans lequel
les données numériques correspondant à l'intensité de signal introduite dans l'algorithme d'apprentissage profond (60) comprennent des informations obtenues en combinant les intensités de signaux du signal de lumière diffusée latéralement, du signal de lumière diffusée vers l'avant et du signal de fluorescence qui ont été obtenues pour chaque cellule.

16. Un appareil d'entraînement pour entraîner un algorithme d'apprentissage profond (50) présentant une structure de réseau neuronal pour l'analyse de cellules contenues dans un échantillon biologique,
l'appareil d'entraînement comprenant une partie de mesure (400) et une partie de traitement (10), dans lequel
la partie de mesure (400) est configurée pour :
faire s'écouler les cellules à travers une cuve à circulation, et introduire, en tant que premières données d'entraînement (75) vers une couche d'entrée (50a) de l'algorithme d'apprentissage profond (50), des données numériques correspondant à des intensités de signaux de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence ; et
la partie de traitement (10) est configurée pour :
introduire, en tant que secondes données d'entraînement (75) vers l'algorithme d'apprentissage profond (50), des informations d'un type d'une cellule qui correspond à la cellule pour laquelle les intensités de signaux ont été obtenues, dans lequel
les données numériques correspondant à l'intensité de signal introduite dans l'algorithme d'apprentissage profond (60) comprennent des informations obtenues en combinant les intensités de signaux du signal de lumière diffusée latéralement, du signal de lumière diffusée vers l'avant et du signal de fluorescence qui ont été obtenues pour chaque cellule.

17. Un programme informatique destiné à analyser des cellules contenues dans un échantillon biologique, à l'aide d'un algorithme d'apprentissage profond (60) présentant une structure de réseau neuronal,
le programme informatique étant configuré pour amener une partie de mesure (400) à exécuter un processus comprenant :
une étape consistant à faire s'écouler les cellules à travers une cuve à circulation, et à obtenir des intensités de signaux de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence ;
le programme informatique étant en outre configuré pour amener une partie de traitement (10) à exécuter un processus comprenant :
une étape consistant à introduire, dans l'algorithme d'apprentissage profond (60), des données numériques correspondant aux intensités de signaux obtenues concernant chacune des cellules individuelles ; et
une étape consistant à, sur la base d'un résultat délivré par l'algorithme d'apprentissage profond (60), déterminer, pour chaque cellule, un type de la cellule pour laquelle les intensités de signaux ont été obtenues, dans lequel
les données numériques correspondant à l'intensité de signal introduite dans l'algorithme d'apprentissage profond (60) comprennent des informations obtenues en combinant les intensités de signaux du signal de lumière diffusée latéralement, du signal de lumière diffusée vers l'avant et du signal de fluorescence qui ont été obtenues pour chaque cellule.

18. Un programme informatique destiné à entraîner un algorithme d'apprentissage profond (50) présentant une structure de réseau neuronal pour l'analyse de cellules contenues dans un échantillon biologique,
le programme informatique étant configuré pour amener une partie de mesure (400) à exécuter un processus comprenant :
une étape consistant à faire s'écouler les cellules à travers une cuve à circulation, et à introduire, en tant que premières données d'entraînement (75) vers une couche d'entrée (50a) de l'algorithme d'apprentissage profond (50), des données numériques correspondant à des intensités de signaux obtenues de signaux lumineux qui sont des signaux obtenus par l'application de lumière sur chacune des cellules individuelles passant à travers la cuve à circulation, les signaux lumineux étant un signal de lumière diffusée latéralement, un signal de lumière diffusée vers l'avant et un signal de fluorescence ; et le programme informatique étant configuré pour amener une partie de traitement (10) à exécuter un processus comprenant : une étape consistant à introduire, en tant que secondes données d'entraînement (75) vers l'algorithme d'apprentissage profond (50), des informations d'un type d'une cellule qui correspond à la cellule pour laquelle les intensités de signaux ont été obtenues, dans lequel
les données numériques correspondant à l'intensité de signal introduite dans l'algorithme d'apprentissage profond (50) comprennent des informations obtenues en combinant les intensités de signaux du signal de lumière diffusée latéralement, du signal de lumière diffusée vers l'avant et du signal de fluorescence qui ont été obtenues pour chaque cellule.
